(19) Europäisches Patentamt
European Patent Office
Office européen des brevets

(11) Numéro de publication : **0 350 414 B1**

# (12) FASCICULE DE BREVET EUROPEEN

(45) Date de publication du fascicule du brevet : **22.04.92 Bulletin 92/17**

(51) Int. Cl.$^5$ : **C08F 220/04,** C08F 8/44, C09K 7/02, C09D 7/12, A61K 7/00

(21) Numéro de dépôt : **89420237.3**

(22) Date de dépôt : **04.07.89**

## (54) Agent épaississant modificateur des caractéristiques rhéologiques de compositions aqueuses chargées et/ou pigmentées, blanches ou colorées.

(30) Priorité : **07.07.88 FR 8809509**

(43) Date de publication de la demande : **10.01.90 Bulletin 90/02**

(45) Mention de la délivrance du brevet : **22.04.92 Bulletin 92/17**

(84) Etats contractants désignés : **AT BE CH DE ES GB GR IT LI NL SE**

(56) Documents cités :
**EP-A- 0 173 109**
**US-A- 4 514 552**
**US-A- 4 600 761**

(73) Titulaire : **Société COATEX, Société Anonyme**
**35 Cours Aristide Briand**
**F-69300 Caluire (FR)**

(72) Inventeur : **Simonet, Benoit**
**Saint Jean des Vignes**
**F-69380 Lozanne (FR)**
Inventeur : **Fabre, Pierre**
**6, Impasse des Jeux de Boules**
**F-69300 Caluire (FR)**
Inventeur : **Laluet, Jacques**
**91, Rue Marinus Berliet**
**F-69008 Lyon (FR)**
Inventeur : **Egraz, Jean-Bernard**
**Impasse Moulin Carron**
**F-69130 Ecully (FR)**

EP 0 350 414 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne un copolymère épaississant associatif hydrosoluble en milieu neutre ou alcalin, ayant la propriété de modifier les caractéristiques rhéologiques des compositions aqueuses chargées et/ou pigmentées, blanches ou colorées.

L'invention concerne également les compositions aqueuses, dont il est souhaitable qu'elles aient un comportement newtonien, chargées et/ou pigmentées, blanches ou colorées, contenant ledit copolymère épaississant associatif, dont l'effet sur le milieu procure simultanément un bon compromis rhéologique à haut et bas cisaillements, se traduisant par de bonnes caractéristiques de pouvoir garnissant et de tension du film après application, tout en gardant une bonne résistance à la coulure.

Dans la description de l'objet de l'invention, l'expression "compositions aqueuses chargées et/ou pigmentées, blanches ou colorées" définit le domaine des suspensions aqueuses de charges et/ou de pigments telles que, par exemple, les compositions de revêtement et plus particulièrement les peintures, les sauces d'enduction, les pâtes d'impression, les produits de finition du cuir, les compositions pour cosmétiques et détergence, les fluides de forage.

En outre, et plus spécialement dans le cas des peintures, le "pouvoir garnissant", caractérise la quantité de peinture déposée par unité de surface, la "résistance à la coulure" définit la capacité d'une peinture à résister à l'écoulement après son application sur un support à protéger, enfin la "tension du film" après application définit la faculté de ladite peinture à niveler les irrégularités d'épaisseur nées de son application sur un support à protéger.

Arrière plan de l'invention.

Pour l'homme de l'art, une composition aqueuse chargée et/ou pigmentée est formée d'une phase liquide qui peut être de l'eau ou un solvant organique miscible à l'eau, ou encore un mélange des deux, d'un polymère en émulsion dans la phase liquide dénommé "liant", des charges et/ou des pigments, d'un agent dispersant des charges et/ou pigments qui peut être un polymère ou copolymère hydrosoluble, des adjuvants aussi divers que des agents de coalescence, des biocides, des antimousses ou autres, enfin d'un agent épaississant qui est un polymère ou copolymère naturel ou de synthèse.

Depuis longtemps, il s'est révélé à l'usage que la présence de l'agent épaississant dans une composition aqueuse chargée et/ou pigmentée était nécessaire pour en modifier les caractéristiques rhéologiques.

Plusieurs espèces d'épaississants pour compositions aqueuses chargées et/ou pigmentées ont déjà été proposées à l'homme de l'art et décrites en abondance dans la littérature spécialisée.

Une première espèce d'agents épaississants pour compositions aqueuses chargées et/ou pigmentées consiste en des dérivés de la cellulose ayant la propriété de viscosifier seulement la phase aqueuse desdites compositions.

Mais le domaine d'application de cette première espèce d'agents épaississants est limité, car des inconvénients se manifestent, perturbant l'utilisateur comme, par exemple, des difficultés de dissolution dans les milieux aqueux, une cinétique d'hydratation souvent lente, une grande sensibilité bactérienne, et aussi une incapacité de faire évoluer, et plus précisément d'ajuster a posteriori la viscosité des compositions aqueuses chargées et/ou pigmentées.

Ainsi, il apparaît que cette première espèce d'agents épaississants provoque au sein des compositions aqueuses chargées et/ou pigmentées, dans lesquelles elle est utilisée, des caractéristiques rhéologiques pseudo-plastiques pouvant être gênantes, telles qu'une viscosité élevée en absence de contrainte mécanique mais qui diminue très fortement sous l'effet d'un cisaillement, ayant pour conséquence un mauvais pouvoir garnissant.

Une autre espèce d'agents épaississants a été proposée pour remédier à certains inconvénients précités de cette première espèce. Cette autre espèce est formée de latex synthétiques qui peuvent être acryliques, dont des descriptions illustratives sont données par exemple dans les brevets tels que FR 2,131,128, FR 2,281,389, US 2,798,053, US 2,985,625 ou encore US 2,958,679.

C'est ainsi qu'est décrit (FR 2,281,389) un polymère réticulé d'anhydride maléique et d'éthylène en présence d'isocyanurate de trialkyle, utilisable comme agent épaississant pour des systèmes aqueux comme les peintures à base de latex synthétiques, les traitements de floculation des minerais, les traitements de coagulation des eaux industrielles ou ménagères.

Ces agents épaississants sont en général des polymères d'acides carboxyliques à fonction éthylénique ou des copolymères de ces mêmes acides et de leurs esters présentés sous forme d'émulsion aqueuse de basse viscosité, du type huile dans eau.

Solubles en milieux aqueux et alcalin, lesdits agents épaississants offrent l'avantage, par rapport aux dérivés cellulosiques précités, d'être plus facilement mis en oeuvre et d'être insensibles à l'attaque bactérienne.

Mais, de même que la première espèce d'agents épaississants, la deuxième espèce possède certains inconvénients tels que provoquer l'épaississement de la seule phase aqueuse desdites compositions, ou encore de procurer à ces compositions des caractéristiques rhéologiques trop pseudo-plastiques qui ne sont pas adaptées à toutes les formulations de peintures.

Plus récemment, une dernière espèce d'agents épaississants hydrosolubles est apparue dans le domaine des compositions aqueuses chargées et/ou pigmentées, agents épaississants classiquement dénommés épaississants associatifs, car ils agissent non seulement par l'augmentation de la viscosité de la phase aqueuse (par solubilisation), mais aussi par la création de liens divers entre le copolymère et certains constituants des compositions, très vraisemblablement par l'apparition d'interactions hydrophobes et de liaisons hydrogènes. L'avantage de ces agents épaississants associatifs, par comparaison avec les agents précités, est d'apporter aux compositions chargées et/ou pigmentées dans lesquelles ils sont mis en oeuvre un comportement moins pseudo-plastique.

En particulier, dans le cas de certaines peintures, l'homme de l'art cherche à obtenir une viscosité sous bas cisaillement qui soit assez faible afin que le film déposé sur le support à protéger ait tendance à bien niveler les irrégularités d'épaisseur dues à l'application (tension du film convenable), ait une viscosité sous haut cisaillement qui soit suffisamment importante afin d'améliorer le pouvoir garnissant et de diminuer les projections quand l'application desdites peintures est faite au rouleau.

Cette dernière espèce d'agents épaississants associatifs s'est développée selon deux familles, celle des épaississants associatifs polyuréthanes et celle des épaississants associatifs acryliques.

Les agents épaississants associatifs polyuréthanes appartenant à la première famille comportent dans leur molécule une ou plusieurs chaînes polyéthers terminées par des groupements hydrophobes, tels que, par exemple, alkyls, aryls, alkylaryls, et sont obtenus par chimie de condensation.

De tels agents sont décrits dans de nombreux brevets, par exemple dans les brevets GB 1,069,735, US 3,770,684, US 4,079,028 et US 4,155,892.

Mais, bien que ces agents procurent aux compositions chargées et/ou pigmentées dans lesquelles ils sont mis en oeuvre des caractéristiques rhéologiques souhaitables, ils sont à l'origine de certains désavantages gênants pour leur utilisateur.

En effet, ces agents se présentent sous une forme visqueuse de manipulation peu aisée, éventuellement en solution dans des mélanges d'eau et de solvant(s), le solvant pouvant être plus ou moins toxique et par là même soumis à une limitation d'usage, ou encore présenter des incompatibilités réactionnelles avec certains constituants des compositions chargées et/ou pigmentées.

Les agents épaississants associatifs acryliques obtenus par polymérisation radicalaire, appartenant à la deuxième famille, hydrosolubles en milieu neutre ou alcalin, sont formés de copolymères préparés en général à partir d'acides carboxyliques éthyléniques, éventuellement d'esters de ces acides et/ou d'autres monomères, et enfin d'au moins un monomère fonctionnel particulier disposant d'une chaîne latérale composée de groupements polyéthers comportant des radicaux terminaux hydrocarbonés hydrophobes.

La nature du monomère fonctionnel particulier s'est révélée être déterminante dans le comportement rhéologique des compositions chargées et/ou pigmentées contenant l'agent épaississant correspondant.

Ainsi, le monomère fonctionnel particulier peut être un acrylate ou méthacrylate d'alcool surfactant (brevets EP 0,013,836 et US 4,384,096), ou peut résulter de l'estérification par un alcool surfactant d'oligomères de l'acide acrylique (brevet US 4,421,902). Ce monomère fonctionnel particulier peut être également un ester oxyéthylé de l'acide crotonique (brevet US 4,569,965), ou encore un hémiester de l'anhydride maléïque (brevet EP 0,248,612), ou bien un éther surfactant de l'alcool allylique (brevet EP 0,216,479).

Ce monomère fonctionnel particulier peut enfin résulter de la condensation d'un alcool surfactant et d'un isocyanate insaturé (brevets US 4,514,552 et US 4,600,761), la présence de groupements uréthanes O-C(O)-NH- sur les chaînes latérales du copolymère apportant un effet bénéfique sur le comportement rhéologique des compositions aqueuses chargées et/ou pigmentées et en particulier les peintures aqueuses.

Le but recherché par l'utilisation des agents épaississants associatifs acryliques était de modifier, dans le sens le plus favorable, les caractéristiques rhéologiques desdites compositions par leur présence, de telle manière qu'elles disposent préférentiellement d'une viscosité maîtrisée simultanément sous haut et bas cisaillements, afin d'avoir un bon pouvoir garnissant, une résistance à la coulure acceptable et une tension du film convenable.

Or, il a été constaté que, parmi les nombreux agents épaississants acryliques associatifs de l'art antérieur:

– certains parvenaient déjà à un profil rhéologique acceptable, mais au prix de doses d'emploi élevées que le formulateur souhaitait voir diminuer,

– les autres, tels que ceux évoqués dans l'US 4,514,552, ne parvenaient pas à maîtriser simultanément les viscosités à haut et bas cisaillements, ou bien tels que ceux évoqués dans l'US 4,600,761 ne parvenaient pas à des compositions sans seuil d'écoulement.

Sommaire de l'invention.

Forte des inconvénients précités, la Demanderesse, poursuivant ses recherches, a trouvé et mis au point un copolymère épaississant associatif hydrosoluble en milieu neutre ou alcalin qui confère aux compositions aqueuses chargées et/ou pigmentées par opposition à l'art antérieur, un bon état de compromis rhéologique à haut et bas cisaillements, se manifestant par un haut pouvoir garnissant et une bonne tension de film tout en gardant une excellente résistance à la coulure, aussi bien pour des compositions blanches que colorées, et ce à des doses d'emploi plus faibles.

Selon l'invention, le copolymère épaississant associatif hydrosoluble en milieu neutre ou alcalin, composé :

a) d'au moins un monomère à insaturation éthylénique disposant d'au moins une fonction carboxylique,

b) d'au moins un autre monomère à insaturation éthylénique démuni de fonction carboxylique,

c) d'au moins un monomère surfactant ayant au moins une fonction uréthane résultant de la réaction d'un isocyanate à insaturation éthylénique avec un composé surfactant possédant une fonction hydroxyle réactive à l'égard du groupement -NCO,

se caractérise en ce que ledit copolymère appartient au groupe constitué par ceux qui, par définition, mis en solution aqueuse à 2% en poids de matière sèche à un pH de 9 obtenue par addition d'ammoniaque et à une température de 20°C, ont une viscosité Brookfield type RVT à 100 tours par minute au plus égale à 220 millipascals.seconde (centipoises).

Description détaillée.

Le copolymère épaississant associatif selon l'invention se distingue de l'art connu par le fait qu'il procure un état de compromis dans lesdites compositions et peintures, conduisant à un équilibre entre les effets extrêmes antérieurement observés.

En d'autres termes, le copolymère épaississant associatif selon l'invention, lorsqu'il est mis en oeuvre dans des compositions aqueuses chargées et/ou pigmentées, génère des milieux dont les caractéristiques rhéologiques générales se rapprochent d'un comportement newtonien, milieux dont la viscosité sous haut cisaillement est augmentée, et dont la viscosité sous bas cisaillement est diminuée par rapport à l'art connu, améliorant en conséquence les caractéristiques de pouvoir garnissant et de tension du film après l'application, tout en maintenant une bonne résistance à la coulure.

Le comportement newtonien des compositions aqueuses contenant le copolymère épaississant associatif selon l'invention est acquis conjointement grâce à l'incorporation dans sa structure d'un monomère surfactant ayant au moins une fonction uréthane et grâce au fait que le poids moléculaire dudit copolymère est très faible, le poids moléculaire étant exprimé par la mesure de la viscosité Brookfield RVT mobiles 1 à 3 à 100 tours par minute d'une solution aqueuse contenant 2% en poids dudit copolymère sec porté à un pH de 9 par addition d'ammoniaque et à une température de 20°C, la limite supérieure de la viscosité Brookfield mesurée dans ces conditions étant au plus de 220 millipascals.seconde (centipoises).

En-dehors de ce domaine conditionnel des viscosités Brookfield telles que précitées, tout copolymère de même structure, mais générant une viscosité supérieure à 220 mPa.s (cP) dans les conditions évoquées pour cette mesure conduit à des compositions aqueuses ayant des caractéristiques rhéologiques perturbées, voire mauvaises quand il est mis en oeuvre comme agent épaississant plus précisément dans le cas des peintures brillantes ou satinées.

Ainsi, comme la Demanderesse a pu le constater, il apparaît d'une manière surprenante que les deux conditions précitées conduisant au copolymère selon l'invention coopèrent pleinement, par la sélection réalisée des poids moléculaires faibles, en créant un état de synergie permettant l'obtention de compositions aqueuses chargées et/ou pigmentées, blanches ou colorées, ayant des caractéristiques rhéologiques se rapprochant d'un comportement newtonien.

Le copolymère épaississant associatif selon l'invention est constitué, comme cela a été antérieurement exprimé, par au moins trois types de monomères.

Le premier type de monomère, qui est un acide carboxylique à insaturation éthylénique, est un composé présentant une liaison éthylénique et au moins un groupe carboxylique ou un groupe anhydride d'acide carboxylique.

Le monomère éthylénique carboxylé peut être choisi parmi les monoacides, tels que l'acide acrylique, méthacrylique, crotonique, isocrotonique, cinnamique, les diacides, tels que l'acide itaconique, fumarique, maléïque, citraconique, les anhydrides d'acides carboxyliques, tels que l'anhydride maléïque et les hémiesters de diacides, tels que les monoesters en $C_1$ à $C_4$, des acides maléïque ou itaconique.

Toutefois, le monomère éthylénique carboxylé est préférentiellement choisi dans le groupe constitué par les acides acrylique, méthacrylique et itaconique.

Le deuxième type de monomère, démuni de fonction carboxylique et qui est à insaturation éthylénique, peut être choisi d'une manière non limitative dans le groupe constitué par les esters d'acides acrylique ou méthacrylique, tels que les acrylates ou méthacrylates de méthyle, éthyle, butyle, 2-éthyl-hexyle, lauryle; les acrylates et méthacrylates d'éthylène glycol, propylène glycol, polyéthylène glycol et polypropylène glycol ainsi que les phosphates et sulfates correspondants, l'acrylonitrile; l'acrylamide, la n-méthylolacrylamide; les acrylates et méthacrylates de diméthylaminoéthyle; l'alcool allylique, l'acétate de vinyle, l'acide acrylamidométhyl propane sulfonique, le styrène, le méthylstyrène.

Cependant, le monomère à insaturation éthylénique de deuxième type est préférentiellement choisi parmi les esters acryliques, tels que les acrylate et méthacrylate d'alkyle en $C_1$ à $C_4$.

Le troisième type de monomère, qui est un monomère surfactant ayant au moins une fonction uréthane, résulte de la réaction d'un isocyanate à insaturation éthylénique avec un composé surfactant possédant une fonction hydroxyle réactive à l'égard du groupement -NCO.

C'est dans la revendication 6 qu'on trouve une réprésentation supplémentaire de l'invention.

L'isocyanate à insaturation éthylénique peut être préparé selon les méthodes bien connues de l'homme de l'art, telles que celles décrites par exemple dans le brevet US 2,718,516.

Cependant, comme ces méthodes de préparation d'isocyanate à insaturation éthylénique sont relativement longues, il est souvent préférable d'utiliser des monoisocyanates à insaturation éthylénique connus tels que, par exemple, le méthacrylate d'isocyanatoéthyle (commercialisé par DOW CHEMICAL COMPANY), les isomères méta ou para de l'alpha-alpha diméthyl isopropyl benzyl isocyanate (commercialisé par AMERICAN CYANAMID CORPORATION).

Toutefois, une méthode d'obtention préférée consiste en l'addition stoechiométrique et goutte à goutte sur un diisocyanate, d'un composé éthylénique présentant un seul hydrogène actif à l'égard du groupement -NCO dans les conditions de réaction choisies.

Comme composé éthylénique, il est possible d'utiliser, par exemple, les acrylates et méthacrylates d'éthylène glycol, de propylène glycol, de polyéthylène glycol et de polypropylène glycol, l'alcool allylique, l'allylamine, la méthallylamine, l'orthoallylphénol.

Comme diisocyanate, il est possible d'utiliser, par exemple, le 1,4 tétraméthylène diisocyanate, le 1,6 hexaméthylène diisocyanate, le 2,2,4 triméthyl 1,6 diisocyanatohexane, le 1,10 décaméthylène diisocyanate, le 4,4 méthylène-bis(isocyanatocyclohexane), le 1,4 cyclohexylène diisocyanate, le 1-isocyanato 3-isocyanatométhyl-3,5,5-triméthylcyclohexane, les m- et p-phénylène diisocyanate, les 2,4 et 2,6 toluène diisocyanate, le xylène diisocyanate, le 4-chloro 1,3-phénylène diisocyanate, le 4,4′-méthylène diphénylisocyanate, le 1,5-naphtalène diisocyanate, le tétrahydronaphtylène diisocyanate.

Le composé surfactant intervenant dans la constitution du monomère surfactant (c) possédant une fonction hydroxyle réactive à l'égard du groupement -NCO de l'isocyanate à insaturation éthylénique répond à la formule générale :

$$R_1 \text{(}0\text{-}R_2\text{)}_n\text{-OH}$$

dans laquelle:

– le motif ($O-R_2$) est l'oxyde d'éthylène, l'oxyde de propylène, l'oxyde de butylène, ou une combinaison de deux au moins de ces groupements oxygénés,

– n, qui représente le nombre moyen de motifs présents dans ledit surfactant, prend une valeur comprise entre 5 et 150,

– le motif $R_1$ est choisi dans le groupe constitué par les structures chimiques hydrocarbonées et/ou aminées, telles que les alkyles aliphatiques ou cycloaliphatiques, les aryls substitués ou non, les polyaryls comportant de 1 à 32 atomes de carbone, les amines secondaires de formule ($R_3$) ($R_4$) N-, dans laquelle $R_3$ et $R_4$ sont des groupements hydrocarbonés comportant 1 à 20 atomes de carbone.

Ainsi, le composé surfactant doté d'une fonction hydroxyle réactive à l'égard du groupement -NCO peut être choisi préférentiellement parmi les dilaurylamines éthoxylées, les octyl et nonyl phénols éthoxylés, les alcools laurique, stéarique, cétylique oxyéthylés, les mono, di et tristyryls phénols éthoxylés, pris seuls ou en mélange.

D'une manière préférentielle, le motif $R_1$ est choisi parmi les chaînes hydrocarbonées en $C_{12}$ à $C_{30}$, le motif $-O-R_2$, parmi les oxydes d'éthylène et/ou de propylène et n dans l'intervalle 15 à 50.

Le copolymère épaississant associatif selon l'invention contient, exprimé en pour cent en poids :

a) de 15 à 75%, et préférentiellement de 30 à 45% de monomère(s) à insaturation éthylénique disposant d'au moins une fonction carboxylique,

b) de 25 à 70%, et préférentiellement de 45 à 60% d'autre(s) monomère(s) à insaturation éthylénique démuni(s) de fonction carboxylique,

c) de 0,5 à 35%, et préférentiellement de 4 à 15% de monomère(s) surfactant(s) ayant au moins une fonction uréthane résultant de la réaction d'un isocyanate à insaturation éthylénique avec un composé surfactant possédant une fonction hydroxyle réactive à l'égard du groupement -NCO,

le total des constituants (a), (b) et (c) étant égal à 100.

Le copolymère épaississant associatif selon l'invention, c'est-à-dire disposant d'une viscosité Brookfield à 100 tours par minute au plus égale à 220 mPa.s (centipoises) dans les conditions précitées, est préparé selon les procédés connus de copolymérisation radicalaire, en solution, en émulsion ou en suspension, du mélange des monomères précités, en présence d'un système catalytique et d'agents de transfert connus, mis en oeuvre selon des quantités appropriées, le poids moléculaire dudit copolymère étant ajusté à l'aide des moyens suivants : température, taux de catalyseur, présence d'un agent de transfert ou tout autre moyen ou combinaison de moyens connus de l'homme de l'art.

Le système catalytique de polymérisation est souhaitablement choisi parmi ceux qui sont hydrosolubles tels que, par exemple, les persulphates de sodium, de potassium, d'ammonium, mis en oeuvre conjointement avec un composé réducteur connu tel que, par exemple, le métabisulfite de sodium.

La quantité de système catalytique de polymérisation peut varier entre 0,1% et 2% en poids de la masse totale des monomères utilisés pour l'obtention du copolymère selon l'invention.

L'agent de transfert est souhaitablement choisi parmi les alkyl-mercaptans tels que, par exemple, l'octanethiol, le décanethiol, le n-dodécanethiol, le t-dodécanethiol.

La quantité d'agent de transfert peut varier entre 0% et 5,0% en poids par rapport à la masse totale des monomères présents.

La température de copolymérisation peut varier entre 30°C et 150°C. Elle est préférentiellement choisie inférieure à la température d'ébullition la plus faible des constituants présents dans les conditions de l'expérience.

Les copolymères selon l'invention développent leur propriété épaississante en milieu alcalin, d'une manière telle que les fonctions carboxyliques présentes soient totalement ou partiellement neutralisées, l'agent de neutralisation étant préférentiellement l'hydroxyde de lithium, de sodium, de potassium, d'ammonium, de calcium, de magnésium, une amine, ou une combinaison de ces agents.

L'invention concerne également les compositions aqueuses chargées et/ou pigmentées contenant le copolymère selon l'invention.

Les compositions aqueuses chargées et/ou pigmentées sont plus particulièrement celles qui, blanches ou colorées, contiennent comme constituants principaux une phase aqueuse, des charges et/ou des pigments, un liant naturel ou synthétique et éventuellement comme constituants secondaires un agent dispersant, des adjuvants aussi divers que des agents de coalescence, des biocides, des tensio-actifs, des anti-mousses ou autres et le copolymère épaississant associatif selon l'invention.

Le copolymère épaississant associatif selon l'invention est introduit dans lesdites compositions à raison de 0,1 à 10%, et préférentiellement à raison de 0,1 à 5,0% et très préférentiellement de 0,4 à 1,5%, quantité exprimée en pour cent en poids sec par rapport à la masse totale de la composition.

En pratique, la phase liquide résultant de la copolymérisation peut être utilisée sous cette forme comme agent épaississant associatif, mais elle peut également être séchée par tous moyens connus pour en éliminer cette phase et isoler le copolymère sous la forme d'une fine poudre et être utilisée sous cette autre forme comme agent épaississant associatif.

Le copolymère épaississant associatif selon l'invention s'applique à des compositions aqueuses chargées et/ou pigmentées telles que les compositions de revêtement, et plus particulièrement les peintures, les sauces d'enduction, les pâtes d'impression, les produits de finition du cuir, les compositions pour cosmétiques et détergents, les fluides de forage.

La portée et l'intérêt de l'invention seront mieux perçus grâce aux exemples suivants.

## Exemple 1

Cet exemple a pour but d'illustrer la préparation d'un monomère surfactant, résultant de la réaction d'un isocyanate à insaturation éthylénique sur un composé surfactant possédant une fonction hydroxyle réactive à l'égard du groupement -NCO.

Pour ce faire, le monomère surfactant a été préparé selon le schéma réactionnel suivant :

**Etape 1**

$$CH_2=CH-CH_2-OH \;+\; OCN-Tol-NCO \;\text{-->}\; CH_2=CH-CH_2-O-\overset{O}{\overset{\|}{C}}-NH-Tol-NCO$$

1 mole 1 mole isocyanate à insaturation éthylénique

(1) + (2) --> (3)

**Etape 2**

$$(3) + C_9H_{19}-Ph-O-(CH_2CH_2-O)_{29}-CH_2CH_2OH \;\text{-->}$$

(4)

$$CH_2=CH-CH_2-O-\overset{O}{\overset{\|}{C}}-NH-Tol-NH-\overset{O}{\overset{\|}{C}}-O-CH_2CH_2-(OCH_2CH_2)_{29}-O-Ph-C_9H_{19}$$

monomère M2

Tol : $C_6H_3-CH_3$

Ph : $C_6H_4$

(1) : source d'insaturation éthylénique

(2) : diisocyanate

(3) : isocyanate à insaturation éthylénique

(4) : composé surfactant (alcool)

M2 : monomère surfactant à insaturation éthylénique

dans lequel (3), qui est l'isocyanate à insaturation éthylénique, réagit sur (4), qui est le composé surfactant.

La préparation a été effectuée selon les étapes suivantes :

Dans un réacteur muni d'une agitation mécanique et d'un séparateur Dean-Stark surmonté d'un réfrigérant, on a placé 154 grammes de nonyl phénol poly(éthylèneoxy)$_{29}$ éthanol et 200 grammes d'heptane. On a alors chauffé le milieu à 90°C pour déshydrater le surfactant. Lorsque la quantité d'eau recueillie dans le séparateur ne variait plus, on a distillé l'heptane, à pression atmosphérique d'abord, puis sous pression réduite (5 mm Hg). Le milieu a alors été refroidi puis maintenu à la température de fusion du surfactant.

Dans un bécher, on a placé 17,4 grammes de toluène diisocyanate, 3 gouttes d'alloocimène (inhibiteur de polymérisation) et 0,24 gramme de dilaurate de dibutylétain (catalyseur). Le bécher a été placé dans un bain eau-glace et agité magnétiquement à 50 tours par minute.

Dans une ampoule de coulée, on a placé 5,8 grammes d'alcool allylique conservé sur tamis moléculaire. On a coulé goutte à goutte en 30 minutes l'alcool allylique sur le toluène diisocyanate, de façon à ce que la température du milieu réactionnel ne dépasse pas 20°C. Le milieu a ensuite été laissé sous agitation jusqu'à ce que le dosage des groupements NCO indique que 50% des fonctions isocyanates ont réagi. Le contenu du bécher a été alors coulé en 10 minutes dans le réacteur contenant l'alcool surfactant fondu. On a laissé ensuite le milieu sous agitation pendant deux heures. Le dosage des groupements NCO a montré alors que toutes les fonctions isocyanates présentes initialement avaient disparu. On a obtenu ainsi quantitativement un monomère

surfactant présentant deux liaisons uréthanes. Ce produit est le monomère M2 du tableau I.

On a répété ce procédé de synthèse en utilisant d'autres isocyanates comportant une insaturation éthylénique et d'autres composés surfactants (alcools) en ajustant les quantités de réactifs, de manière à respecter la stoechiométrie de la réaction. On a obtenu ainsi une série de monomères surfactants cités dans le tableau I sous les références M1 à M88.

Toutefois, dans le cas des monomères M51 à M70 faisant usage d'isocyanates à insaturation éthylénique commerciaux, l'étape 1 du schéma précité n'existe pas.

## TABLEAU I

### Préparation de monomères surfactants

| référence du monomère surfactant | source d' insaturation éthylénique | diisocyanate | alcool surfactant R | alcool surfactant n |
|---|---|---|---|---|
| M1 | alcool allylique | TDI | NP | 50 |
| M2 | alcool allylique | TDI | NP | 30 |
| M3 | alcool allylique | TDI | NP | 17 |
| M4 | alcool allylique | TDI | C12 | 23 |
| M5 | alcool allylique | TDI | C12 | 17 |
| M6 | alcool allylique | TDI | C12 | 11 |
| M7 | alcool allylique | TDI | C16-18 | 25 |
| M8 | alcool allylique | TDI | C16-18 | 33 |
| M9 | alcool allylique | TDI | C16-18 | 50 |
| M10 | alcool allylique | TDI | Distyrylphénol | 15 |
| M11 | MAEG | TDI | NP | 50 |
| M12 | MAEG | TDI | NP | 30 |
| M13 | MAEG | TDI | NP | 17 |
| M14 | MAEG | TDI | C12 | 23 |
| M15 | MAEG | TDI | C12 | 17 |
| M16 | MAEG | TDI | C12 | 11 |
| M17 | MAEG | TDI | C16-18 | 25 |
| M18 | MAEG | TDI | C16-18 | 33 |
| M19 | MAEG | TDI | C16-18 | 50 |
| M20 | MAEG | TDI | Distyrylphénol | 15 |
| M21 | MA (PEG)10 | TDI | NP | 50 |
| M22 | MA (PEG)10 | TDI | NP | 30 |
| M23 | MA (PEG)10 | TDI | NP | 17 |
| M24 | MA (PEG)10 | TDI | C12 | 23 |
| M25 | MA (PEG)10 | TDI | C12 | 17 |
| M26 | MA (PEG)10 | TDI | C12 | 11 |
| M27 | MA (PEG)10 | TDI | C16-18 | 25 |
| M28 | MA (PEG)10 | TDI | C16-18 | 33 |
| M29 | MA (PEG)10 | TDI | C16-18 | 50 |
| M30 | MA (PEG)10 | TDI | Distyrylphénol | 15 |

## TABLEAU I

(suite)

| référence du monomère surfactant | source d'insaturation éthylénique | diisocyanate | alcool surfactant R | n |
|---|---|---|---|---|
| M31 | Allylamine | TDI | NP | 50 |
| M32 | Allylamine | TDI | NP | 30 |
| M33 | Allylamine | TDI | NP | 17 |
| M34 | Allylamine | TDI | C12 | 23 |
| M35 | Allylamine | TDI | C12 | 17 |
| M36 | Allylamine | TDI | C12 | 11 |
| M37 | Allylamine | TDI | C16-18 | 25 |
| M38 | Allylamine | TDI | C16-18 | 33 |
| M39 | Allylamine | TDI | C16-18 | 50 |
| M40 | Allylamine | TDI | Distyrylphénol | 15 |
| M41 | AEG | TDI | NP | 50 |
| M42 | AEG | TDI | NP | 30 |
| M43 | AEG | TDI | NP | 17 |
| M44 | AEG | TDI | C12 | 23 |
| M45 | AEG | TDI | C12 | 17 |
| M46 | AEG | TDI | C12 | 11 |
| M47 | AEG | TDI | C16-18 | 25 |
| M48 | AEG | TDI | C16-18 | 33 |
| M49 | AEG | TDI | C16-18 | 50 |
| M50 | AEG | TDI | Distyrylphénol | 15 |
| M51 | m-TMI | / | NP | 50 |
| M52 | m-TMI | / | NP | 30 |
| M53 | m-TMI | / | NP | 17 |
| M54 | m-TMI | / | C12 | 23 |
| M55 | m-TMI | / | C12 | 17 |
| M56 | m-TMI | / | C12 | 11 |
| M57 | m-TMI | / | C16-18 | 25 |
| M58 | m-TMI | / | C16-18 | 33 |
| M59 | m-TMI | / | C16-18 | 50 |
| M60 | m-TMI | / | Distyrylphénol | 15 |

## TABLEAU I

(suite)

| référence du monomère surfactant | source d' insaturation éthylénique | diisocyanate | alcool surfactant R | n |
|---|---|---|---|---|
| M61 | IEM | / | NP | 50 |
| M62 | IEM | / | NP | 30 |
| M63 | IEM | / | NP | 17 |
| M64 | IEM | / | C12 | 23 |
| M65 | IEM | / | C12 | 17 |
| M66 | IEM | / | C12 | 11 |
| M67 | IEM | / | C16-18 | 25 |
| M68 | IEM | / | C16-18 | 33 |
| M69 | IEM | / | C16-18 | 50 |
| M70 | IEM | / | Distyrylphénol | 15 |
| M71 | alcool allylique | IPDI | NP | 50 |
| M72 | alcool allylique | IPDI | NP | 30 |
| M73 | alcool allylique | IPDI | NP | 17 |
| M74 | alcool allylique | IPDI | C12 | 23 |
| M75 | alcool allylique | IPDI | C12 | 17 |
| M76 | alcool allylique | IPDI | C12 | 11 |
| M77 | alcool allylique | IPDI | C16-18 | 25 |
| M78 | alcool allylique | IPDI | C16-18 | 33 |
| M79 | alcool allylique | IPDI | C16-18 | 50 |
| M80 | alcool allylique | IPDI | Distyrylphénol | 15 |
| M81 | MAEG | IPDI | NP | 50 |
| M82 | AEG | IPDI | NP | 50 |
| M83 | MA (PEG)10 | IPDI | NP | 50 |
| M84 | allylamine | IPDI | NP | 50 |
| M85 | MAEG | TDI | (C12)2-N | 50 |
| M86 | AEG | TDI | (C12)2-N | 50 |
| M87 | MA (PEG)10 | TDI | (C12)2-N | 50 |
| M88 | allylamine | TDI | (C12)2-N | 50 |

MAEG = méthacrylate d'éthylène glycol
MA (PEG)10 = méthacrylate de poly(éthylèneoxy)9-éthanol
TDI = toluène diisocyanate
R = radical hydrocarboné
n = nombre moyen de groupements oxyde d'éthylène
NP = radical nonyl phénol
C12 = radical lauryle
C16-18 = radical céto-stéaryle
AEG = acrylate d'éthylène glycol
m-TMI = méta-isopropènyl diméthylbenzyl isocyanate
IEM = isocyanatoéthyl méthacrylate
IPDI = isophorone diisocyanate
(C12)2-N = radical dilaurylamine

Exemple 2

Cet exemple illustre la préparation d'un copolymère épaississant associatif selon l'invention en mettant en oeuvre le monomère surfactant M2 du tableau I.

Dans ce but, on a tout d'abord préparé une pré-émulsion de monomères en ajoutant dans l'ordre sous agitation les composés ci-dessous, selon les quantités pondérales indiquées :

| | |
|---|---|
| - eau bipermutée | 155,00 g |
| - laurylsulfate éther de Na (poudre) | 1,75 g |
| - n-dodécanethiol | 0,95 g |
| - acrylate d'éthyle | 147,50 g |
| - monomère surfactant M2 | 25,00 g |
| - acide méthacrylique | 100,00 g |

Dans un réacteur muni d'un réfrigérant et d'une agitation mécanique, on a placé, selon les quantités pondérales indiquées :

| | |
|---|---|
| - eau bipermutée | 506,00 g |
| - laurylsulfate éther de Na (poudre) | 2,35 g |

On a amené le contenu du réacteur à 68°C. On a ajouté alors 1 gramme de persulfate d'ammonium en solution dans 5 grammes d'eau et 0,1 gramme de métabisulfite de sodium en solution dans 5 grammes d'eau. On a introduit alors en continu pendant deux heures la pré-émulsion de monomères et on a maintenu la température dans le réacteur à 75°C. Le milieu réactionnel a ensuite été porté à 80°C pendant une heure, puis refroidi. On a obtenu ainsi une émulsion à 28,7% en poids de matière sèche du copolymère épaississant associatif W du tableau II. Une solution aqueuse à 2% dudit copolymère sec portée à un pH de 9 par addition d'ammoniaque avait une viscosité de 120 mPa.s (cP) (Brookfield RVT, mobile 2, 100 tr/mn, 20°C).

Ce mode de préparation a été répété en utilisant différents monomères surfactants à insaturation éthylénique tels que préparés selon l'exemple 1. On a obtenu ainsi une série de copolymères épaississants associatifs selon l'invention, se présentant sous l'aspect d'une émulsion (références A à AR dans le tableau II).

Dans ce tableau, et conformément à l'invention, les copolymères épaississants associatifs mis en solution aqueuse à 2% en poids de matière sèche portée à un pH de 9 par addition d'ammoniaque et à une température de 20°C, ont tous une viscosité Brookfield (type RVT) à 100 tours par minute au plus égale à 220 mPa.s (cP).

## TABLEAU II

### Synthèses de copolymères selon l'invention

| référence du copolymère | référence du monomère MS | % monomères MS | AMA | AEt | % nDDT | viscosité solution 2% à pH 9 100 tr/mn |
|---|---|---|---|---|---|---|
| A | M1 | 9,2 | 36,7 | 54,1 | 0,37 | 90 |
| B | M4 | 4,6 | 39,1 | 56,3 | 0,37 | 54 |
| C | M24 | 9,2 | 36,7 | 54,1 | 0,26 | 55 |
| D | M26 | 9,2 | 36,7 | 54,1 | 0,26 | 120 |
| E | M4 | 9,2 | 36,7 | 54,1 | 0,57 | 63 |
| F | M14 | 11,4 | 35,8 | 52,8 | 0,18 | 116 |
| G | M4 | 11,4 | 35,8 | 52,8 | 0,47 | 100 |
| H | M14 | 9,2 | 36,7 | 54,1 | 0,28 | 72 |
| I | M6 | 9,4 | 37,8 | 52,8 | 0,87 | 47 |
| J | M6 | 9,4 | 37,8 | 52,8 | 0,66 | 85 |
| K | M5 | 9,2 | 36,7 | 54,1 | 0,61 | 82 |
| L | M7 | 9,2 | 36,7 | 54,1 | 0,83 | 71 |
| M | M8 | 9,2 | 36,7 | 54,1 | 0,60 | 41 |
| N | M8 | 9,2 | 36,7 | 54,1 | 0,49 | 57 |
| P | M6 | 9,2 | 36,7 | 54,1 | 0,66 | 87 |
| Q | M8 | 9,2 | 36,7 | 54,1 | 0,39 | 90 |
| R | M4 | 9,2 | 36,7 | 54,1 | 0,50 | 70 |
| S | M8 | 9,2 | 36,7 | 54,1 | 0,50 | 58 |
| T | M3 | 9,2 | 36,7 | 54,1 | 0,40 | 156 |
| U | M9 | 9,2 | 36,7 | 54,1 | 0,40 | 132 |
| V | M3 | 9,2 | 36,7 | 54,1 | 0,60 | 65 |
| W | M2 | 9,2 | 36,7 | 54,1 | 0,35 | 120 |
| X | M1 | 9,2 | 36,7 | 54,1 | 0,59 | 70 |
| Y | M2 | 9,2 | 36,7 | 54,1 | 0,50 | 77 |
| Z | M1 | 9,2 | 36,7 | 54,1 | 0,40 | 88 |
| AA | M14 | 4,3 | 36,1 | 59,6 | 0,26 | 95 |
| AB | M14 | 6,9 | 37,6 | 55,5 | 0,28 | 59 |
| AC | M14 | 13,8 | 34,8 | 51,4 | 0,28 | 73 |
| AD | M14 | 11,5 | 35,7 | 52,7 | 0,18 | 99 |
| AE | M14 | 9,2 | 36,7 | 54,1 | 0,13 | 208 |
| AF | M34 | 9,2 | 36,7 | 54,1 | 0,50 | 45 |
| AG | M10 | 9,2 | 36,7 | 54,1 | 0,50 | 132 |
| AH | M14 | 10,0 | 30,0 | 60,0 | 0,28 | 67 |
| AI | M14 | 10,0 | 20,0 | 70,0 | 0,28 | 41 |
| AJ | M14 | 10,0 | 40,0 | 50,0 | 0,28 | 82 |
| AK | M14 | 15,0 | 55,0 | 30,0 | 0,28 | 87 |
| AL | M14 | 10,0 | 45,0 | 45,0 | 0,28 | 78 |
| AM | M14 | 15,0 | 15,0 | 70,0 | 0,28 | 30 |
| AN | M14 | 14,3 | 23,8 | 61,9 | 0,28 | 37 |
| AP | M14 | 20,0 | 30,0 | 50,0 | 0,28 | 91 |
| AQ | M14 | 5,0 | 55,0 | 40,0 | 0,28 | 65 |
| AR | M54 | 9,2 | 36,7 | 54,1 | 0,15 | 58 |

AMA = acide méthacrylique
AEt = acrylate d'éthyle
MS = monomère surfactant
nDDT = n-dodécanethiol

Exemple 3

Cet exemple a pour but de comparer des copolymères épaississants associatifs, objets de l'invention provenant du tableau II, à des agents épaississants associatifs commercialisés, appartenant à l'art antérieur.

Parmi les agents épaississants associatifs de l'art connu, trois d'entre eux, réputés être parmi les meilleurs, ont été sélectionnés en vue de cette étude comparative.

Le premier, dénommé alpha dans la suite du texte, est le PRIMAL RM5, commercialisé par la société ROHM & HAAS.

Le deuxième, dénommé beta dans la suite du texte, est le VISCALEX VG2, commercialisé par la société ALLIED COLLOIDS.

Le troisième, dénommé gamma dans la suite du texte, est le MOWILITH LDM 7000, commercialisé par la société HOECHST.

Pour ce faire, on a préparé une série de peintures aqueuses brillantes et blanches dans lesquelles seuls la nature et le taux de l'agent épaississant associatif changeaient.

Les formulations de ces peintures ont été faites à partir d'une formule d'orientation proposée par des producteurs de liants aux formulateurs pour le liant mis en oeuvre et couramment utilisé dans ce type de peinture.

Les quantités des constituants desdites peintures, hormis les agents épaississants objets de la comparaison, ont été exprimées en grammes, tandis que la quantité d'épaississant a été exprimée en pourcentage de polymère sec par rapport au total de chaque formulation.

Les formulations utilisées ont été rassemblées dans le tableau III-A et les résultats dans le tableau III-B.

## TABLEAU III-A

| | | Epaississant | | | | | | |
|---|---|---|---|---|---|---|---|---|
| | | alpha | beta | gamma | H | H | B | G |
| référence peinture blanche | | 3-1 | 3-2 | 3-3 | 3-4 | 3-5 | 3-6 | 3-7 |
| Formule peinture blanche: | | | | | | | | |
| coalescent : Propylène glycol | 50,20 | | | | | | | |
| eau | 42,00 | | | | | | | |
| dispersant : Coatex DR 3 (a) | 4,20 | | | | | | | |
| bactéricide: Mergal K6N (b) | 2,50 | | | | | | | |
| antimousse : Nopco NDW (c) | 0,80 | | | | | | | |
| pigment : TiO2 RHD2 (d) | 196,60 | | | | | | | |
| liant : Primal HG 74 (e) | 568,50 | | | | | | | |
| coalescent : méthoxybutanol | 25,10 | | | | | | | |
| coalescent : Texanol (f) | 36,20 | | | | | | | |
| ammoniaque (28%) | 3,00 | | | | | | | |
| **épaississant (% sec/total formule)** | | 0,82 | 0,82 | 0,82 | 0,82 | 0,62 | 0,82 | 0,82 |
| antimousse : Nopco NDW | 0,80 | | | | | | | |
| ammoniaque (28%) | qsp pH 8,7 | | | | | | | |
| eau | qsp total 1000 | | | | | | | |
| total | 1000,00 | | | | | | | |

(a): commercialisé par la société Coatex (France)
(b): commercialisé par la société Omya (France)
(c): commercialisé par la société Henkel (RFA)
(d): commercialisé par la société Tioxide (GB)
(e): commercialisé par la société Rohm and Haas (USA)
(f): commercialisé par la société Eastman Chemicals (USA)

Pour une exploitation des résultats du tableau III-B, il est souhaitable de définir les méthodes et/ou moyens mis en oeuvre pour obtenir lesdits résultats.

**Viscosité 24 heures.**

Cette viscosité a été mesurée d'une manière systématique 24 heures après la réalisation de chaque peinture blanche.

La mesure a été effectuée au moyen d'un viscosimètre Brookfield RVT à 20°C à l'aide d'un mobile choisi de manière à obtenir une déviation de l'aiguille comprise dans l'intervalle de graduation 15 à 80. La valeur obtenue est représentative de la viscosité en pot desdites peintures (viscosité sous bas cisaillement).

**Viscosité apparente.**

Cette mesure a été effectuée à l'aide d'un rhéomètre RHEOMAT 30 de marque CONTRAVES.

Pour ce faire, on a placé une petite quantité de peinture dans l'entrefer (50 $\mu$m) d'un mobile HS 50, et on a enregistré la courbe contrainte de cisaillement ($\tau$) en fonction du gradient de vitesse (D). La viscosité apparente est le rapport de $\tau$/D pour D = 17700 s-1.

Le cisaillement à 17700 s-1 est représentatif des hauts cisaillements rencontrés lors de l'application des peintures à la brosse ou au rouleau. La viscosité apparente mesurée apparaît donc comme un moyen d'observer ce que sera le comportement de la peinture sous la brosse ou le rouleau.

**Limite d'écoulement.**

Cette mesure a également été effectuée à l'aide d'un rhéomètre RHEOMAT 30 de la marque CONTRAVES mettant en oeuvre un mobile DIN 25.

Pour réaliser cette mesure de limite d'écoulement, la peinture a d'abord été soumise à un cisaillement important (environ 500 s-1) afin de la destructurer. Puis on a enregistré la courbe : contrainte de cisaillement en fonction du gradient de vitesse (D), ce gradient variant de 0 à 5 s-1. L'intersection de la tangente à la partie basse de la courbe avec l'axe des contraintes de cisaillement donne la limite d'écoulement.

**Tension du film.**

La tension du film après application définit la faculté des peintures à niveler les irrégularités d'épaisseur nées de leur application sur un support à protéger. Pour en effectuer la mesure, on a pratiqué une simulation des irrégularités en déposant sur une plaque de verre cinq paires de cordons de section initiale rectangulaire, les dépôts de chaque paire étant séparés par un intervalle de 2 millimètres. Les épaisseurs des cordons sont de 0,25 millimètre, 0,50 millimètre, 1 millimètre, 2 millimètres et 4 millimètres. La plaque a été maintenue horizontale. On a noté après séchage le nombre de paires de dépôts dont les cordons se sont joints. La tension du film la meilleure est donc de 5, c'est-à-dire toutes paires de cordons jointes, et la moins bonne 0, c'est-à-dire aucune paire jointe.

**Résistance à la coulure.**

La résistance à la coulure définit la capacité d'une peinture à résister à l'écoulement après son application sur un support à protéger.

Pour en effectuer la mesure, on a déposé sur une plaque de verre à l'aide d'une jauge 10 cordons de 6 millimètres de large et d'épaisseurs comprises entre 75 et 300 microns, par pas de 25 microns, les différents dépôts étant séparés par un intervalle de 2 millimètres. La plaque est alors placée verticalement, les dépôts les plus épais étant placés en bas. On a noté après séchage le nombre de bandes non jointes. La résistance à la coulure la meilleure est donc de 10 et la moins bonne de 0.

Les deux dernières mesures de tension du film et de résistance à la coulure ont été effectuées selon la norme ASTM D 2801-69.

**Brillance.**

La mesure de la brillance, dont la technique est bien connue de l'homme de métier, a été effectuée au moyen d'un brillancemètre de marque ERICHSEN, sur film sec après 24 heures.

**Application.**

Après l'application à la brosse de la peinture sur un support type kraft, l'opérateur a jugé selon son savoir-faire trois qualités d'application qui sont la brossabilité, la tension du film et le pouvoir garnissant selon les notations suivantes :

- brossabilité
  - A = excellent
  - B = bon
  - C = moyen
  - D = difficile à appliquer
  - E = très difficile à appliquer

la brossabilité traduisant la sensation plus ou moins agréable que l'applicateur éprouve lors de l'application du film.

- tension du film
  - A = excellent
  - B = bon
  - C = tend assez bien
  - D = corde un peu
  - E = corde beaucoup
- pouvoir garnissant
  - A = garnit très bien
  - B = garnit bien
  - C = garnit assez bien
  - D = garnit peu
  - E = garnit mal

Tous les résultats relatifs aux tests précités ont été regroupés dans le tableau III-B.

# TABLEAU III-B

| | Epaississant (% sec / total formule) | | | | | | |
|---|---|---|---|---|---|---|---|
| | alpha 0,82 | beta 0,82 | gamma 0,82 | H 0,82 | H 0,62 | E 0,82 | G 0,82 |
| référence peinture blanche | 3-1 | 3-2 | 3-3 | 3-4 | 3-5 | 3-6 | 3-7 |
| pH | 8,7 | 8,7 | 8,8 | 8,7 | 8,7 | 8,7 | 8,7 |
| viscosité Brookfield : 10 tr/mn | 2800 | 11800 | 11600 | 7200 | 6000 | 4500 | 6800 |
| 24 heures mPa.s (cP) 100 tr/mn | 1600 | 2650 | 3300 | 3880 | 3440 | 2780 | 3840 |
| viscosité apparente (mPa.s) | 186 | 130 | 191 | 331 | 222 | 209 | 241 |
| Limite d'écoulement (Pa) | 1,30 | 12,50 | 11,00 | 3,20 | 2,20 | 1,30 | 2,10 |
| tension du film | 3 | 2 | 2 | 2 | 3 | 3 | 3 |
| résistance à la coulure | 2 | 9 | 9 | 9 | 7 | 8 | 9 |
| brillance (%) 20° | 68 | 67 | 71 | 67 | 68 | 65 | 67 |
| 24 heures 60° | 85 | 85 | 87 | 86 | 85 | 84 | 86 |
| 85° | 95 | 94 | 96 | 95 | 95 | 94 | 94 |
| application: brossabilité | D | D | C | C | D | A | D |
| tension du film | C | B | D | B | D | D | D |
| pouvoir garnissant | C | B | C | A | A | D | A |

Ce tableau III-B permet de constater que la viscosité apparente des peintures épaissies à l'aide des copolymères associatifs selon l'invention est toujours supérieure à 200 mPa.s, alors que la viscosité apparente des peintures épaissies à l'aide des agents épaississants actuellement commercialisés et faisant partie de l'art antérieur se situe toujours au-dessous de 200 mPa.s, ceci à taux d'épaississant constant.

Dans le cas de la peinture 3-4 (objet de l'invention), la viscosité apparente est supérieure de 70% à la meilleure viscosité apparente des peintures de l'art connu.

Dans le cas des peintures 3-1 à 3-3, qui constituent l'art connu, il est remarqué que :

– pour la peinture 3-1, la viscosité à 24 heures est trop faible et conduit à une résistance à la coulure médiocre,

– pour les peintures 3-2 et 3-3, cette viscosité à 24 heures est trop élevée et conduit à une limite d'écoulement trop importante se traduisant à l'application par une mauvaise tension du film (effet de cordage).

Dans le cas des peintures 3-4 et 3-5, objets de l'invention, la diminution du taux de l'agent épaississant (24% d'abaissement par rapport à l'art antérieur) permet de maintenir la viscosité apparente à un niveau favorable, toujours supérieur à 200 mPa.s tout en diminuant la viscosité à 24 heures et la limite d'écoulement permettant, sans nuire au pouvoir garnissant, d'améliorer la tension du film ainsi que la brossabilité.

Quant aux peintures 3-6 et 3-7 faisant également objet de l'invention, elles offrent les caractéristiques rhéologiques et d'applications supérieures aux caractéristiques de l'art antérieur.

Exemple 4

Cet exemple a pour but de comparer des copolymères épaississants associatifs, objets de l'invention provenant du tableau II, à des agents épaississants associatifs commercialisés, appartenant à l'art antérieur.

Comme agents épaississants associatifs de l'art connu, on a retenu les trois agents alpha, beta et gamma définis dans l'exemple 3.

Les formulations de ces peintures ont été faites à partir d'une formule d'orientation proposée par le producteur de liants aux formulateurs pour le liant mis en oeuvre et couramment utilisé dans ce type de peinture.

Les quantités des constituants desdites peintures, hormis les agents épaississants objets de la comparaison, ont été exprimées en grammes, tandis que la quantité d'épaississant a été exprimée en pourcentage de polymère sec par rapport au total de chaque formulation.

Les formulations utilisées ont été rassemblées dans le tableau IV-A et les résultats dans le tableau IV-B.

## TABLEAU IV-A

| | | Epaississant | | | | |
|---|---|---|---|---|---|---|
| | | gamma | beta | alpha | H | H |
| référence peinture blanche | | 4-1 | 4-2 | 4-3 | 4-4 | 4-5 |
| Formule peinture blanche: | | | | | | |
| eau | 25,00 | | | | | |
| dispersant : Coatex DR3 (a) | 4,00 | | | | | |
| antimousse : Tego Foamex 1488 (b) | 0,80 | | | | | |
| bactéricide: Mergal K6N (c) | 2,20 | | | | | |
| coalescent : propylène glycol | 25,00 | | | | | |
| pigment : $TiO_2$ RHD2 (d) | 210,00 | | | | | |
| liant : Mowilith LDM 7770 (e) | 600,00 | | | | | |
| coalescent : propylène glycol | 75,00 | | | | | |
| coalescent : Texanol (f) | 15,00 | | | | | |
| ammoniaque (28%) | 4,40 | | | | | |
| **épaississant (%sec/total formule)** | | **0,58** | **0,58** | **0,58** | **0,58** | **0,43** |
| eau | qsp total 1024 | | | | | |
| ammoniaque (28%) | qsp pH 8,7 | | | | | |
| total | 1024,00 | | | | | |

(a): commercialisé par la société Coatex (France)
(b): commercialisé par la société Tego Chemie (RFA)
(c): commercialisé par la société Omya (France)
(d): commercialisé par la société Tioxide (GB)
(e): commercialisé par la société Hoechst (RFA)
(f): commercialisé par la société Eastman Chemicals

Les tests répertoriés dans le tableau IV-B ont été réalisés conformément aux définitions données dans l'exemple 3.

Tous les résultats relatifs aux tests précités ont été regroupés dans le tableau IV-B.

## TABLEAU IV-B

| | Epaississant (% sec / total formule) | | | | |
|---|---|---|---|---|---|
| | gamma 0,58 | beta 0,58 | alpha 0,58 | H 0,58 | H 0,43 |
| référence peinture blanche | 4-1 | 4-2 | 4-3 | 4-4 | 4-5 |
| pH | 8,7 | 8,7 | 8,7 | 8,7 | 8,7 |
| viscosité Brookfield : 10 tr/mn<br>24 heures mPa.s (cP) 100 tr/mn | 4800<br>1440 | 1600<br>740 | 2600<br>1280 | 6000<br>2840 | 3400<br>1800 |
| viscosité apparente (mPa.s) | 149 | 86 | 122 | 214 | 161 |
| limite d'écoulement (Pa) | 1,00 | 0,50 | 0,10 | 2,30 | 0,80 |
| tension du film | 3 | 5 | 5 | 4 | 4 |
| résistance à la coulure | 5 | 0 | 1 | 7 | 7 |
| brillance (%) 20°<br>24 heures 60°<br>85° | 59<br>73<br>75 | 60<br>75<br>78 | 64<br>79<br>81 | 64<br>81<br>84 | 61<br>80<br>87 |
| application: brossabilité<br>tension du film<br>pouvoir garnissant | D<br>C<br>C | B<br>D<br>B | B<br>A<br>D | A<br>A<br>A | D<br>A<br>D |

Le tableau IV-B permet de constater, en harmonie avec le tableau III-B, la supériorité évidente de l'épaississant associatif H selon l'invention. En effet, à taux d'épaississants identique, la viscosité apparente de la peinture 4-4 (objet de l'invention) est de 40% supérieure à la meilleure viscosité apparente des peintures relatives à l'art antérieur (4-1 à 4-3).

Dans le cas des peintures 4-4 et 4-5 qui constituent l'objet de l'invention, la diminution de 26% du taux d'agent épaississant (peinture 4-5) conduit à une peinture dont les caractéristiques de rhéologie et d'application sont toujours supérieures à celles des peintures relatives à l'art connu (4-1 à 4-3).

## Exemple 5

Cet exemple a pour but de comparer des copolymères épaississants associatifs, objets de l'invention provenant du tableau II, à des agents épaississants associatifs commercialisés, appartenant à l'art antérieur.

Comme agents épaississants associatifs de l'art connu, on a retenu les trois agents alpha, beta et gamma définis dans l'exemple 3.

Les formulations de ces peintures ont été faites à partir d'une formule d'orientation proposée par le producteur de liants aux formulateurs pour le liant mis en oeuvre et couramment utilisé dans ce type de peinture.

Les quantités des constituants desdites peintures, hormis les agents épaississants objets de la comparaison, ont été exprimées en grammes tandis que la quantité d'épaississant a été exprimée en pourcentage de polymère sec par rapport au total de chaque formulation.

Les formulations utilisées ont été rassemblées dans le tableau V-A et les résultats des tests dans le tableau V-B.

## TABLEAU V-A

| | | Epaississant | | | | | |
|---|---|---|---|---|---|---|---|
| | | alpha | beta | beta | gamma | gamma | H |
| référence peinture blanche | | 5-1 | 5-2 | 5-3 | 5-4 | 5-5 | 5-6 |
| **Formule peinture blanche:** | | | | | | | |
| coalescent : propylène glycol | 24,30 | | | | | | |
| eau | 31,80 | | | | | | |
| dispersant : Coatex DR3 (a) | 4,00 | | | | | | |
| bactéricide: Mergal K6N (b) | 1,50 | | | | | | |
| antimousse : Dyk 073 (c) | 1,00 | | | | | | |
| pigment : TiO2 RHD2 (d) | 230,30 | | | | | | |
| ammoniaque (28%) | 2,50 | | | | | | |
| coalescent : éthyl diglycol | 24,50 | | | | | | |
| coalescent : butyl diglycol | 24,50 | | | | | | |
| liant : Néocryl XK 76 (e) | 580,60 | | | | | | |
| **épaississant (% sec /total formule)** | | 1,10 | 0,50 | 1,10 | 0,40 | 1,10 | 1,10 |
| eau | qsp total 1000 | | | | | | |
| antimousse : Dyk 073 | 1,00 | | | | | | |
| ammoniaque (28%) | qsp pH 8,7 | | | | | | |
| total | 1000,00 | | | | | | |

(a): commercialisé par la société Coatex (France)
(b): commercialisé par la société Omya (France)
(c): commercialisé par la société Dyk Chemie (RFA)
(d): commercialisé par la société Tioxide (GB)
(e): commercialisé par la société Polyvinyl Chemie (Pays-Bas)

Les tests répertoriés dans le tableau V-B ont été réalisés conformément aux définitions qui en ont été données dans l'exemple 3.

Tous les résultats relatifs aux tests précités ont été regroupés dans le tableau V-B.

## TABLEAU V-B

| | Epaississant (% sec / total formule) | | | | | |
|---|---|---|---|---|---|---|
| | alpha 1,10 | beta 0,50 | beta 1,10 | gamma 0,40 | gamma 1,10 | M 1,10 |
| référence peinture blanche | 5-1 | 5-2 | 5-3 | 5-4 | 5-5 | 5-6 |
| pH | 8,7 | 8,7 | 8,7 | 8,7 | 8,7 | 8,7 |
| viscosité Brookfield : 10 tr/mn | 5000 | 4000 | 9500 | 3400 | 13300 | 3600 |
| 24 heures mPa.s (cP) 100 tr/mn | 1720 | 1040 | 2570 | 760 | 2960 | 2320 |
| viscosité apparente (mPa.s) | 172 | 65 | 153 | 61 | 186 | 245 |
| limite d'écoulement (Pa) | 2,00 | 6,00 | 6,30 | 4,20 | 11,20 | 1,50 |
| tension du film | 3 | 1 | 3 | 3 | 0 | 3 |
| résistance à la coulure | 7 | 8 | 9 | 7 | 9 | 6 |
| brillance 20° | 54 | 53 | 56 | 52 | 57 | 54 |
| 24 heures 60° | 76 | 75 | 78 | 73 | 77 | 76 |
| 85° | 84 | 87 | 88 | 84 | 89 | 88 |
| application: brossabilité | C | D | C | D | C | A |
| tension du film | C | D | B | C | B | A |
| pouvoir garnissant | A | B | D | B | D | A |

Le tableau V-B montre que, en conformité avec les tableaux III-B et IV-B, la peinture formulée avec l'agent épaississant associatif selon l'invention (5-6) à un taux de 1,1% par rapport au poids total de la formule, possède une viscosité apparente supérieure de 30% à la meilleure viscosité apparente des peintures relatives à l'art antérieur (5-1, 5-3 et 5-5) pour un même taux en sec d'agent épaississant.

De plus, dans le cas des peintures 5-3 et 5-5 relatives à l'art connu, les viscosités à 24 heures (en pot) sont trop élevées, engendrant des limites d'écoulement trop importantes affectant la tension du film.

Dans le but de combattre ce phénomène, la diminution du taux d'agent épaississant (références 5-2 et 5-4) provoque une baisse raisonnable de la viscosité à 24 heures, mais une chute considérable de la viscosité apparente, ayant pour conséquence l'abaissement inacceptable du pouvoir garnissant.

Dès lors, à travers les exemples 3, 4 et 5, il apparaît que les agents épaississants associatifs acryliques selon l'invention apportent aux peintures dans lesquelles ils sont mis en oeuvre des caractéristiques rhéologiques et d'application qui sont toujours supérieures à celles des peintures épaissies à l'aide des agents épaississants commerciaux réputés être parmi les meilleurs.

Car ces agents procurent aux peintures, non seulement un pouvoir garnissant important (viscosité apparente élevée), mais aussi un excellent compromis entre la tension du film et la résistance à la coulure, caractéristiques antagonistes par nature.

De plus, ces agents peuvent être mis en oeuvre à des taux inférieurs à ceux des épaississants de l'art antérieur, tout en conservant aux peintures les contenant des caractéristiques de rhéologie et d'application meilleures que celles des peintures formulées avec les agents épaississants de l'art antérieur.

Enfin, les agents épaississants selon l'invention se révèlent, à travers les exemples, procurer aux peintures les contenant une régularité des caractéristiques rhéologiques et d'application non constatée dans les peintures formulées au moyen des agents épaississants de l'art connu.

## Exemple 6

Cet exemple a pour but d'illustrer l'influence du poids moléculaire de l'agent épaississant associatif sur son aptitude à être mis en oeuvre dans une formulation dans une peinture aqueuse brillante.

Cet exemple a plus particulièrement pour objet de prouver que le poids moléculaire dudit agent épaississant doit être inférieur à une valeur limite pour qu'il conserve son aptitude à être mis en oeuvre dans lesdites peintures, cette valeur limite exprimée en viscosité Brookfield étant au plus égale à 220 mPa.s (cP) pour une solution aqueuse à 2% de l'agent épaississant sec portée à un pH de 9 par addition d'ammoniaque, à une température de 20°C, pour une vitesse de 100 tours par minute et un mobile permettant une déviation de l'index comprise entre 15 et 80 au moment de la mesure.

Dans cet exemple, la mesure de la viscosité Brookfield RVT relative au poids moléculaire de chaque agent épaississant associatif testé est effectuée, non seulement à une concentration de 2%, mais aussi à une concentration de 1% de l'agent épaississant sec selon la méthode de mesure décrite dans le brevet US 4,514,552 afin de disposer d'un moyen de mesure commun permettant de comparer les agents épaississants selon l'invention aux agents épaississants de l'art antérieur décrits dans ledit brevet.

Dans ce but, des peintures ont été préparées en conformité quantitative et qualitative à l'essai 3-5 de l'exemple 3, c'est-à-dire avec le même liant qui est le PRIMAL HG 74 de la société ROHM & HAAS.

Les peintures 6-1 et 6-3 contenaient des agents épaississants de l'art connu, et les peintures 6-5 et 6-6 contenaient des agents épaississants selon l'invention, lesdits agents épaississants ayant été mis en oeuvre à raison de 0,62% en produit sec par rapport au total de la formule.

Les peintures 6-2 et 6-4 contenaient des agents épaississants de l'art connu, mais selon des taux plus faibles.

Les résultats relatifs aux peintures précitées et aux tests effectués sur lesdites peintures, tels qu'ils ont été définis dans l'exemple 3, ont été regroupés dans le tableau VI.

## TABLEAU VI

Sélection des poids moléculaires adaptés à la formulation des peintures sur la formule de l'exemple 3

| | | 6-1 | 6-2 | 6-3 | 6-4 | 6-5 | 6-6 |
|---|---|---|---|---|---|---|---|
| référence peinture blanche | | 6-1 | 6-2 | 6-3 | 6-4 | 6-5 | 6-6 |
| référence du polymère | | art antérieur | art antérieur | art antérieur | art antérieur | AB | H |
| nature du monomère | | alc allyl. TDI C12-23 OE | alc allyl. TDI C12-23 OE | MAEG TDI C12-23 OE | MAEG TDI C12-23 OE | MAEG TDI C12-23 OE | MAEG TDI C12-23 OE |
| viscosité 2% mPa.s (cP) | 10 tr/mn | 4800 | 4800 | 750 | 750 | 200 | 40 |
| | 100 tr/mn | 1380 | 1380 | 500 | 500 | 208 | 72 |
| viscosité 1% mPa.s (cP) | 10 tr/mn | 800 | 800 | 220 | 220 | 40 | 20 |
| | 100 tr/mn | 244 | 244 | 172 | 172 | 70 | 52 |
| % sec d'épaississant/total | | 0,62 | 0,25 | 0,62 | 0,17 | 0,62 | 0,62 |
| pH | | 8,6 | 8,6 | 8,6 | 8,7 | 8,6 | 8,7 |
| viscosité Brookfield 24 heures mPa.s (cP) | 10 tr/mn | 27600 | 3000 | 13400 | 2500 | 7000 | 6000 |
| | 100 tr/mn | 5250 | 950 | 5100 | 830 | 3100 | 3440 |
| viscosité apparente (mPa.s) | | 154 | 64 | 240 | 58 | 200 | 222 |
| limite d'écoulement (Pa) | | 23,00 | 2,80 | 7,90 | 2,50 | 2,50 | 2,20 |
| tension du film | | 0 | 3 | 2 | 3 | 3 | 3 |
| résistance à la coulure | | 9 | 9 | 9 | 7 | 8 | 7 |
| brillance (%) 24 heures | 20° | 64 | 60 | 63 | 59 | 65 | 68 |
| | 60° | 85 | 82 | 81 | 80 | 83 | 85 |
| | 85° | 89 | 89 | 88 | 87 | 91 | 95 |
| application: | brossabilité | C | C | C | C | A | B |
| | tension du film | E | B | D | C | B | D |
| | pouvoir garnissant | C | E | A | E | A | A |

alc allyl. = alcool allylique
MAEG = méthacrylate d'éthylène glycol
TDI = toluène diisocyanate
C12 23 OE = lauryl poly(éthylèneoxy)22-éthanol

Ce tableau permet les observations suivantes :

Les peintures 6-1 et 6-3 (relatives à l'art antérieur) ont des viscosités à 24 heures très élevées, qui engendrent des limites d'écoulement inacceptables se traduisant par une très mauvaise tension du film (cordage fortement marqué), bien que la viscosité apparente desdites peintures ne soient pas affectée par les agents épaississants mis en oeuvre.

Les formulations de peintures 6-5 et 6-6 (relatives à l'invention), qui disposent de viscosités apparentes proches de celles mesurées dans le cas de l'art antérieur, se différencient de l'art antérieur par des viscosités à 24 heures et des limites d'écoulement beaucoup plus faibles, qui favorisent grandement la tension du film, critère essentiel pour l'application desdites peintures.

Les formulations 6-2 (à comparer à 6-1) et 6-4 (à comparer à 6-3), qui concernent l'art antérieur, ont été l'objet d'un abaissement du taux d'agent épaississant afin de diminuer les viscosités à 24 heures et les limites d'écoulement excessives pour améliorer la tension du film. Mais, à travers les résultats, il apparaît que la diminution du taux d'épaississant provoque une chute rédhibitoire de la viscosité apparente et donc du pouvoir garnissant.

Enfin, comme on peut le constater dans le brevet US 4,514,552, les épaississants qui s'y trouvent décrits ont une viscosité, en solution aqueuse, à un taux de 1% à 10 tours par minute supérieure ou égale à 178 mPa.s (cP), alors que la viscosité des solutions des copolymères selon l'invention dans les mêmes conditions est, comme indiquée dans le tableau VI, au plus de 40 nillipascals.seconde (centipoises).

Dès lors, il est confirmé que les poids moléculaires moyens des agents épaississants ne doivent pas dépasser une valeur limite, représentée par la viscosité mesurée selon les conditions précitées à un taux de 2% dudit agent, qui'est de 220 mPa.s (cP) à 100 tours par minute, pour que les agents épaississants, qui sont objets de l'invention, confèrent simultanément aux peintures dans lesquelles ils sont mis en oeuvre un bon compromis rhéologique à haut et bas cisaillements, se traduisant simultanément par un bon pouvoir garnissant et une excellente tension du film, critères jamais atteints pour les peintures formulées à l'aide de ce type d'agents épaississants appartenant à l'art antérieur.

<u>Exemple 7</u>

Cet exemple a pour but de confirmer l'influence du poids moléculaire de l'agent épaississant sur son aptitude à être mis en oeuvre dans des formulations de peintures aqueuses brillantes à base, non plus de PRIMAL HG 74 comme dans l'exemple 6, mais à base de NEOCCRYL XK 76 commercialisé par POLYVINYL CHEMIE.

Les peintures testées ont été préparées en conformité quantitative et qualitative avec l'essai 5-1 de l'exemple 5, à l'exception du type de l'agent épaississant et du taux auquel il a été mis en oeuvre dans lesdites peintures.

Les peintures 7-1 et 7-3 (de l'art antérieur) et 7-5 et 7-6 (objets de l'invention) ont mis en oeuvre des épaississants différents mais selon le même taux.

Les peintures 7-2 et 7-4 (de l'art antérieur) ont mis en oeuvre des épaississants différents à des taux plus faibles que pour les autres peintures.

Les résultats relatifs aux peintures précitées et aux tests effectués sur lesdites peintures tels qu'ils ont été définis dans l'exemple 3 ont été regroupés dans le tableau VII.

## TABLEAU VII

Sélection des poids moléculaires adaptés à la formulation des peintures sur la formule de l'exemple 5

| référence peinture blanche: | 7-1 | 7-2 | 7-3 | 7-4 | 7-5 | 7-6 |
|---|---|---|---|---|---|---|
| référence du polymère | art antérieur | art antérieur | art antérieur | art antérieur | AE | H |
| nature du monomère | alc allyl.<br>TDI<br>C12-23 OE | alc allyl.<br>TDI<br>C12-23 OE | MAEG<br>TDI<br>C12-23 OE | MAEG<br>TDI<br>C12-23 OE | MAEG<br>TDI<br>C12-23 OE | MAEG<br>TDI<br>C12-23 OE |
| viscosité 2% 10 tr/mn | 4800 | 4800 | 750 | 750 | 200 | 40 |
| mPa.s (cP) 100 tr/mn | 1380 | 1380 | 500 | 500 | 208 | 72 |
| viscosité 1% 10 tr/mn | 800 | 800 | 220 | 220 | 40 | 20 |
| mPa.s (cP) 100 tr/mn | 244 | 244 | 172 | 172 | 70 | 52 |
| % sec d'épaississant/total | 1,10 | 0,39 | 1,10 | 0,32 | 1,10 | 1,10 |
| pH | 8,6 | 8,6 | 8,6 | 8,7 | 8,6 | 8,7 |
| viscosité Brookfield 24 heures 10 tr/mn | 59000 | 4500 | 16500 | 4200 | 4500 | 3600 |
| mPa.s (cP) 100 tr/mn | 13200 | 1200 | 6650 | 1050 | 2860 | 2320 |
| viscosité apparente (mPa.s) | 275 | 69 | 361 | 71 | 271 | 245 |
| limite d'écoulement (Pa) | non mesurable | 2,50 | 11,70 | 3,40 | 2,50 | 1,50 |
| tension du film | 0 | 3 | 1 | 3 | 3 | 3 |
| résistance à la coulure | 9 | 9 | 9 | 7 | 8 | 6 |
| brillance (%) 20° | 55 | 51 | 54 | 50 | 53 | 54 |
| 24 heures 60° | 72 | 71 | 74 | 72 | 75 | 76 |
| 85° | 84 | 82 | 87 | 85 | 89 | 88 |
| application: brossabilité | E | D | C | D | A | A |
| tension du film | E | B | D | C | B | A |
| pouvoir garnissant | A | E | D | E | A | A |

alc allyl. = alcool allylique
MAEG = méthacrylate d'éthylène glycol
TDI = toluène diisocyanate
C12 23 OE = lauryl poly(éthylèneoxy)22-éthanol

Ce tableau conduit aux observations suivantes. De même que dans l'exemple 6, les épaississants mis en oeuvre dans les peintures 7-1 et 7-3 leur confèrent une viscosité à 24 heures et une limite d'écoulement très élevées, se traduisant par une très mauvaise tension du film (cordage fortement marqué), bien que la viscosité apparente desdites formulations ne soit pas affectée par les agents épaississants mis en oeuvre.

Ces mêmes agents épaississants utilisés à taux plus faible (7-2 et 7-4) conduisent à des viscosités à 24

heures et des limites d'écoulement acceptables, améliorant la tension du film mais détruisant la viscosité apparente et donc le pouvoir garnissant au niveau de l'application.

Les agents épaississants objets de l'invention répondant à la limite sélective selon l'invention des poids moléculaires, confèrent aux formulations de peintures dans lesquelles ils sont mis en oeuvre un excellent compromis rhéologique à haut et bas cisaillements, se traduisant simultanément par un bon pouvoir garnissant et une bonne tension du film, comme cela a déjà été constaté dans l'exemple 6.

Exemple 8

Cet exemple a pour but de montrer la supériorité des épaississants associatifs selon l'invention par rapport aux épaississants de l'art antérieur, du point de vue de leur compatibilité à l'égard des pigments colorés, minéraux ou organiques se présentant sous l'aspect d'une pâte pigmentaire.

Par compatibilité à l'égard des pigments, on entend l'ensemble des effets constatés suivants :

– l'absence d'augmentation sensible de la viscosité de la peinture après l'adjonction de la pâte pigmentaire,
– l'absence de détérioration de la tension du film de la peinture colorée,
– l'absence de phénomène de flottation de pigments, se traduisant par leur remontée à la surface du pot de peinture.

Par pâte pigmentaire, on entend toute suspension concentrée de pigments minéraux ou organiques introduite dans une peinture blanche pour la colorer.

Les pâtes pigmentaires mises en oeuvre ont été décrites dans le tableau VIII-A et sont toutes couramment utilisées dans la profession de la peinture.

Ainsi, les peintures blanches présentant l'un au moins des deux inconvénients suivants :

– viscosité en pot (c'est-à-dire viscosité Brookfield à 24 heures) et limite d'écoulement trop élevées (ayant pour conséquence une mauvaise tension du film : cas des essais 3-2, 5-3, 6-1, 6-3, 7-1 et 7-3),
– viscosité apparente trop faible (peu de pouvoir garnissant : cas des essais 3-2, 4-2, 4-3, 5-2, 5-4, 6-2, 6-4, 7-2 et 7-4),

ont été éliminées des tests de coloration.

Au contraire, des peintures blanches manifestant un bon compromis rhéologique à haut et bas cisaillements (se traduisant par un bon pouvoir garnissant et une bonne tension du film) ont été soumises aux tests de coloration.

Pour ce faire, des peintures répondant aux critères de sélection précités, choisies parmi les exemples 3 à 5, ont été colorées par addition, sous agitation mécanique, de 5 parties en poids de pâte pigmentaire pour 100 parties en poids de peinture blanche, cette quantité ayant été choisie arbitrairement.

## TABLEAU VIII-A

Références des pâtes pigmentaires

| pâte pigmentaire N°: | nature | référence commerciale | société |
|---|---|---|---|
| 1 | oxyde de fer jaune | Telochrome TC 21 BJ 603 | Telosud |
| 2 | oxyde de fer rouge | Telochrome TC 21 BR 317 | Telosud |
| 3 | jaune de chrome | Telochrome TC 21 BJ 655 | Telosud |
| 4 | bleu de phtalocyanine | Luconyl 6900 | BASF |
| 5 | vert de phtalocyanine | Vert 8J | Astra |
| 6 | rouge dinitraniline | Rouge 2J | Astra |
| 7 | noir de carbone | Noir V | Astra |
| 8 | jaune hansa | Jaune 10J | Astra |

La viscosité Brookfield à 24 heures des peintures colorées a été systématiquement mesurée dans le but de constater toute augmentation sensible de viscosité qui serait néfaste à la qualité de la peinture et qui traduirait une incompatibilité entre l'agent épaississant mis en oeuvre et la pâte pigmentaire.

Tous les résultats relatifs à cette étude ont été regroupés dans le tableau VIII-B et concernent la viscosité Brookfield à 24 heures, l'éventuelle flottation des pigments et la qualité du rendu de couleur.

## TABLEAU VIII-B

Compatibilité épaississants / pâtes pigmentaires

| | Liant | Neocryl XK76 | Neocryl XK76 | Primal HG74 | Primal HG74 | Mowilith LDM7770 | Mowilith LDM7770 | Mowilith LDM7770 |
|---|---|---|---|---|---|---|---|---|
| | Epaississant | H | alpha | H | alpha | H | alpha | gamma |
| | Dose (a) | 1,10 | 1,10 | 0,82 | 0,82 | 0,58 | 0,58 | 0,58 |
| | Référence peinture blanche | 5-6 | 5-1 | 3-4 | 3-1 | 4-4 | 4-3 | 4-1 |
| | | viscosité Brookfield mPa.s (cP) 10tr/mn 24 heures 100 tr/mn | | | | | | |
| peinture blanche | | 3600<br>2320 | 5000<br>1720 | 7200<br>3800 | 2800<br>1440 | 6000<br>2840 | 2600<br>1280 | 4800<br>1440 |
| Pâte N°: | | | | | | | | |
| 1 | | 4000<br>2320 | 5600 *<br>2080 $ | 7600<br>4300 | 2800<br>1600 | 6800 *<br>3440 $ | 2800 *<br>1520 | 4400 *<br>1480 |
| 2 | | 3000<br>1960 | 5200 *<br>1960 | 6800<br>3600 $ | 2800<br>1400 | 6400 *<br>2880 | 2600 *<br>1320 | 4400<br>1400 |
| 3 | | 3600<br>1960 | 9200<br>2600 | 5600<br>3080 $ | 3400<br>1560 | 5600<br>2600 | 3600<br>1680 | 6000<br>1720 |
| 4 | | 5600<br>2640 | 13200 *<br>3280 | 7200<br>3720 | 10400 *<br>2320 | 4800<br>1840 | 4800<br>1840 | 7200 *<br>2360 |
| 5 | | 4000<br>1920 | 12400<br>2720 | 6000<br>2880 | 6000 *<br>1960 | 4000<br>2080 | 3400<br>1520 | 7200<br>1960 |
| 6 | | 3200<br>1760 | 10400<br>2720 | 3600<br>2200 | 10000<br>2080 | 3200<br>1480 $ | 4800<br>1440 | 6400<br>1680 |
| 7 | | 16000<br>3400 | 20400<br>3640 | 11400<br>3880 $ | 15600<br>2920 | 5200<br>2360 $ | 6000<br>2040 | 10800<br>2600 |
| 8 | | 4000<br>2400 $ | 11200<br>3000 | 8400<br>4700 $ | 4800<br>1840 | 8000<br>3560 $ | 4800<br>1840 | 6800<br>2160 |
| augmentation maximale visco 10 tr | | 2000 | 8200 | 1200 | 7600 | 2000 | 3400 | 2400 |
| moyenne variation visco 10 tr | | +250 | +4600 | -1000 | +2400 | -600 | +1200 | +1200 |

(a) : % sec / total formule * : flottation des pigments $ : excellent rendu de la couleur

De ce tableau ressortent les observations marquantes suivantes :

– dans le cas des peintures colorées à l'aide de la pâte pigmentaire n° 7, les résultats se révèlent médiocres, sauf dans un cas (peinture blanche 4-4, colorée avec la pâte pigmentaire n° 7),

– a l'exception des peintures formulées au moyen de la pâte pigmentaire n° 7, les peintures colorées et épaissies à l'aide de l'agent épaississant selon l'invention manifestent :

* très peu de cas de flottation de pigments,
* un rendu de couleur au moins toujours équivalent et souvent meilleur que celui obtenu avec les peintures contenant les épaississants de l'art antérieur, à liants et pâtes pigmentaires identiques,
* enfin et surtout une remarquable absence d'augmentation rédhibitoire de la viscosité Brookfield à 24 heures.

Pour illustrer la remarquable absence d'augmentation rédhibitoire de cette viscosité, le tableau VIII-B donne, dans les deux dernières lignes :

– le maximum d'augmentation de la viscosité Brookfield à 10 tours par minute par rapport à la peinture blanche,

– la moyenne en valeur algébrique de la variation de cette viscosité.

Les calculs ont été faits en excluant les résultats obtenus avec la pâte pigmentaire n° 7 qui se sont révélés en général très médiocres quel que soit l'épaississant mis en oeuvre, comme cela a déjà été exprimé.

Ainsi, les maxima d'augmentation et les valeurs moyennes de variation de viscosité montrent que seules les peintures colorées contenant les épaississants selon l'invention ne sont pas sujettes à une augmentation rédhibitoire de viscosité par rapport à la peinture blanche d'origine.

## Revendications

1. Copolymère épaississant associatif hydrosoluble en milieu neutre ou alcalin pour compositions aqueuses chargées et/ou pigmentées, blanches ou colorées composé :

a) d'au moins un monomère à insaturation éthylénique disposant d'au moins une fonction carboxylique,

b) d'au moins un autre monomère à insaturation éthylénique démuni de fonction carboxylique,

c) d'au moins un monomère surfactant ayant au moins une fonction uréthane résultant de la réaction d'un isocyanate à insaturation éthylénique avec un composé surfactant, possédant une fonction hydroxyle réactive à l'égard du groupement -NCO,

caractérisé en ce que ledit copolymère appartient au groupe constitué par ceux qui, par définition, mis en solution aqueuse à 2% en poids de matière sèche portée à un pH de 9 par addition d'ammoniaque et à une température de 20°C, ont une viscosité Brookfield à 100 tours par minute au plus égale à 220 mPa.s (cP).

2. Copolymère selon la revendication 1, caractérisé en ce que le monomère éthylénique (a) possédant au moins une fonction carboxylique est choisi dans le groupe constitué par les monoacides acrylique, méthacrylique, crotonique, cinnamique, les diacides itaconique, fumarique, maléïque, citraconique, l'anhydride maléïque et les hémiesters de diacides en $C_1$ à $C_4$.

3. Copolymère selon les revendications 1 ou 2, caractérisé en ce que le monomère éthylénique carboxylé est préférentiellement l'acide acrylique, l'acide méthacrylique ou l'acide itaconique.

4. Copolymère selon l'une quelconque des revendications 1 à 3, caractérisé en ce que le monomère à insaturation éthylénique (b) démuni de fonction carboxylique est choisi dans le groupe constitué par les acrylates ou méthacrylates de méthyle, éthyle, butyle, 2-éthyl-hexyle, lauryle; les acrylates et méthacrylates d'éthylène glycol, propylène glycol, polyéthylène glycol et polypropylène glycol ainsi que les phosphates et sulfates correspondants, l'acrylonitrile, l'acrylamide, la n-méthylolacrylamide, les acrylates et méthacrylates de diméthylaminoéthyle, l'alcool allylique, l'acétate de vinyle, l'acide acrylamidométhyl propane sulfonique, le styrène, le méthylstyrène.

5. Copolymère selon l'une quelconque des revendications 1 à 4, caractérisé en ce que le monomère à insaturation éthylénique (b) est préférentiellement choisi parmi les acrylates et méthacrylates en $C_1$ à $C_4$.

6. Copolymère selon l'une quelconque des revendications 1 à 5, caractérisé en ce que l'isocyanate à insaturation éthylénique intervenant dans la constitution du monomère surfactant (c) résulte de la réaction d'un diisocyanate sur un composé éthylénique comportant une fonction réactive à l'égard du groupement -NCO.

7. Copolymère selon la revendication 6, caractérisé en ce que le diisocyanate est choisi dans le groupe constitué par le 1,4 tétraméthylène diisocyanate, le 1,6 hexaméthylène diisocyanate, le 2,2,4 triméthyl 1,6 diisocyanatohexane, le 1,10 décaméthylène diisocyanate, le 4,4 méthylène-bis(isocyanatocyclohexane), le 1,4 cyclohexylène diisocyanate, le 1-isocyanato 3-isocyanatométhyl-3,5,5-triméthylcyclohexane, les m- et p-phénylène diisocyanate, les 2,4 et 2,6 toluène diisocyanate, le xylène diisocyanate, le 4-chloro 1,3-phénylène diisocyanate, le 4,4'-méthylène diphénylisocyanate, le 1,5-naphtalène diisocyanate, le tétrahydronaphtylène

diisocyanate.

8. Copolymère selon la revendication 6, caractérisé en ce que le composé éthylénique comportant une fonction réactive à l'égard du groupement -NCO est choisi dans le groupe constitué par les acrylates et méthacrylates d'éthylène glycol, de propylène glycol, polyéthylène glycol et de polypropylène glycol, l'alcool allylique, l'allylamine, la méthallylamine, l'orthoallylphénol.

9. Copolymère selon l'une quelconque des revendications 1 à 8, caractérisé en ce que le composé surfactant intervenant dans la constitution du monomère surfactant (c) répond à la formule :

$$R_1 \text{-}(O\text{-}R_2)_n\text{-}OH$$

dans laquelle :

– le motif ($O\text{-}R_2$) est l'oxyde d'éthylène, l'onde de propylène, l'oxyde de butylène, ou une combinaison de deux au moins de ces groupements oxygénés,

– n, qui représente le nombre moyen de motifs présents dans ledit composé surfactant, prend une valeur comprise entre 5 et 150,

– le motif $R_1$ comportant 1 à 32 atomes de carbone est choisi dans le groupe constitué par les structures chimiques hydrocarbonées et/ou aminées, telles que les alkyles aliphatiques ou cycloaliphatiques, les aryls substitués ou non, les polyaryls, les amines secondaires de formule ($R_3$)($R_4$)N-, dans laquelle $R_3$ et $R_4$ sont des groupements hydrocarbonés, comportant 1 à 20 atomes de carbone.

10. Copolymère selon la revendication 9, caractérisé en ce que, d'une manière préférentielle, le motif $R_1$ est choisi parmi les chaînes hydrocarbonées en $C_{12}$ à $C_{30}$, le motif -O-$R_2$, parmi les oxydes d'éthylène et/ou de propylène et n dans l'intervalle 15 à 50.

11. Copolymère selon l'une des revendications 9 ou 10, caractérisé en ce que le composé surfactant est préférentiellement choisi parmi les dilaurylamines éthoxylées, les octyl et nonyl phénols éthoxylés, les alcools laurique, stéarique, cétylique oxyéthylés, les mono, di et tristyryls phénols éthoxylés, pris seuls ou en mélange.

12. Copolymère selon l'une quelconque des revendications 1 à 11, caractérisé en ce qu'il contient, exprimé en pour cent en poids :

a) de 15 à 75%, et préférentiellement de 30 à 45% de monomère(s) à insaturation éthylénique disposant d'au moins une fonction carboxylique,

b) de 25 à 70%, et préférentiellement de 45 à 60% d'autre(s) monomère(s) à insaturation éthylénique démuni de fonction carboxylique,

c) de 0,5 à 35%, et préférentiellement de 4 à 15% de monomère(s) surfactant(s) ayant au moins une fonction uréthane résultant de la réaction d'un isocyanate à insaturation éthylénique avec un composé surfactant possédant une fonction hydroxyle réactive à l'égard du groupement -NCO.

13. Copolymère selon l'une quelconque des revendications 1 à 12, caractérisé en ce qu'il est au moins partiellement neutralisé.

14. Copolymère selon la revendication 13, caractérisé en ce que l'agent de neutralisation est préférentiellement l'hydroxyde de lithium, de sodium, de potassium, d'ammonium, de calcium, de magnésium ou une amine, pris seuls ou en combinaison.

15. Compositions aqueuses chargées et/ou pigmentées, caractérisées en ce qu'elles contiennent le copolymère défini dans les revendications 1 à 14.

16. Compositions aqueuses chargées et/ou pigmentées selon la revendication 15, caractérisées en ce que, blanches ou colorées, elles contiennent comme constituants principaux une phase aqueuse, des charges et/ou pigments, un liant naturel ou synthétique, le copolymère épaississant associatif selon les revendications 1 à 14, et éventuellement comme constituants secondaires un agent dispersant, des additifs aussi divers que des agents de coalescence, des biocides, des tensio-actifs, des anti-mousses.

17. Compositions aqueuses selon l'une ou l'autre des revendications 15 ou 16, caractérisées en ce que le copolymère épaississant y est introduit à raison de 0,1% à 10%, préférentiellement de 0,1% à 5%, et très préférentiellement de 0,4% à 1,5% en poids sec par rapport à la masse totale desdites compositions.

18. Applications du copolymère épaississant selon l'une quelconque des revendications 1 à 17 aux compositions aqueuses de revêtement, peintures aqueuses, sauces d'enduction, pâtes d'impression, produits de finition du cuir, compositions cosmétiques, détergents et fluides de forage.

**Patentansprüche**

1. Verdickendes, assoziatives und in neutralen oder alkalischen Lösungen wasserlösliches Copolymeres für wäßrige, gefüllte und/oder pigmentierte, weiße oder gefärbte Zusammensetzungen:

a) aus wenigstens einem ethylenisch ungesättigten Monomeren mit wenigstens einer Carboxylfunktion,

b) aus wenigstens einem anderen ethylenisch ungesättigten Monomeren ohne eine Carboxylfunktion,

c) aus wenigstens einem oberflächenaktiven Monomer mit wenigstens einer Urethanfunktion, erhalten durch Reaktion eines ethylenisch ungesättigten Isocyanats mit einer oberflächenaktiven Verbindung, die eine mit der -NCO-Gruppe reaktionsfähige Hydroxylgruppe hat,

dadurch gekennzeichnet, daß das Copolymere zu der Gruppe von Polymeren gehört, die, per Definition, als wäßrige Lösung von 2 Gew.-% der Trockensubstanz durch den Zusatz von Ammoniak auf einen pH-Wert von 9 und auf eine Temperatur von 20°C eingestellt, eine Brookfield-Viskosität bei 100 Umdrehungen pro Minute von höchstens 220 mPa x s (cP) haben.

2. Copolymer nach Anspruch 1, dadurch gekennzeichnet, daß das ethylenische Monomere (a), das wenigstens eine Carboxylfunktion hat, aus der Gruppe von einbasischer Acryl-, Methacryl-, Croton- und Zimtsäure, zweibasischer Itacon-, Fumar-, Malein- und Citraconsäure, Maleinsäureanhydrid und den Halbestern der zweibasischen $C_1$- bis $C_4$-Säuren ausgewählt ist.

3. Copolymer nach einem der Ansprüche 1 oder 2, dadurch gekennzeichnet, daß das carboxylische, ethylenische Monomere vorzugsweise Acryl-, Methacryl- oder Itaconsäure ist.

4. Copolymer nach einem der Ansprüche 1 bis 3, dadurch gekennzeichnet, daß das ethylenisch ungesättigte Monomere (b) ohne Carboxylfunktion aus der Gruppe von Methyl-, Ethyl-, Butyl-, 2-Ethylhexyl-, Laurylacrylaten oder -methacrylaten; den Acrylaten und -methacrylaten von Ethylenglykol-, Propylenglykol-, Polyethylenglykol-, Polypropylenglykol sowie den entsprechenden Phosphaten und Sulfaten, Acrylnitril, Acrylamid, N-Methylolacrylamid, den Acrylaten und -methacrylaten von Dimethylethylamin , Allylalkohol, Vinylacetat, Acrylamidomethylpropansulfonsäure, Styrol und Methylstyrol ausgewählt ist.

5. Copolymer nach einem der Ansprüche 1 bis 4, dadurch gekennzeichnet, daß das ethylenisch ungesättigte Monomere (b) vorzugsweise aus $C_1$ bis $C_4$-Acrylaten und -methacrylaten ausgewählt ist.

6. Copolymer nach einem der Ansprüche 1 bis 5, dadurch gekennzeichnet, daß das ethylenisch ungesättigte Isocyanat, das in die Konstitution des oberflächenaktiven Monomeren (c) eingeht, durch Reaktion eines Diisocyanats mit einer ethylenischen Verbindung mit einer gegenüber der -NCO-Gruppe reaktionsfähigen Gruppe erhalten wurde.

7. Copolymer nach Anspruch 6, dadurch gekennzeichnet, daß das Diisocyanat aus der Gruppe, bestehend aus 1,4-Tetramethylendiisocyanat, 1,6-Hexamethylendiisocyanat, 2,2,4-Trimethyl-1,6-diisocyanatohexan, 1,10-Decamethylendiisocyanat, 4,4-Methylen-bis(isocyanatocyclohexan), 1,4-Cyclohexylendiisocyanat, 1-Isocyanato-3-Isocyanatomethyl-3,5,5-trimethylcyclohexan, m- und p-Phenylendiisocyanat, 2,4- und 2,6-Toluoldiisocyanat, Xyloldiisocyanat, 4-Chlor-1,3-phenylendiisocyanat, 4,4′-Methylendiphenylisocyanat, 1,5-Naphtalindiisocyanat und Tetrahydronaphtylendiisocyanat ausgewählt ist.

8. Copolymer nach Anspruch 6, dadurch gekennzeichnet, daß die ethylenische Verbindung mit einer gegenüber der -NCO-Gruppe reaktionsfähigen Gruppe aus der Gruppe der Acrylate und -methacrylate von Ethylenglykol, Propylenglykol, Polyethylenglykol und Polypropylenglykol, Allylalkohol, Allylamin, Methallylamin und Orthoallylphenol ausgewählt ist.

9. Copolymer nach einem der Ansprüche 1 bis 8, dadurch gekennzeichnet, daß die oberflächenaktive Verbindung, die in die Konstitution des oberflächenaktiven Monomeren (c) eingeht, der Formel:

$$R_1 - (O - R_2)_n - OH$$

entspricht, in der

- die Struktureinheit ($O-R_2$) Ethylenoxid, Propylenoxid, Butylenoxid, oder eine Kombination von wenigstens zwei dieser sauerstoffhaltigen Gruppen ist,
- n, der den Mittelwert der Anzahl der Struktureinheiten, die in der oberflächenaktiven Verbindung enthalten sind, darstellt, einen Wert zwischen 5 und 150 einnimmt,
- der Rest $R_1$ mit 1 bis 32 Kohlenstoffatomen aus der Gruppe der kohlenwasserstoffhaltigen und/oder aminhaltigen chemischen Verbindungen, wie aliphatische oder cycloaliphatische Alkyle, ggfs. substituierte Aryle, Polyaryle, sekundäre Amine der Formel $(R_3)(R_4)N$-, in der $R_3$ und $R_4$ Kohlenwasserstoffreste mit 1 bis 20 Kohlenstoffatomen sind, ausgewählt ist.

10. Copolymer nach Anspruch 9, dadurch gekennzeichnet, daß, die Struktureinheit $R_1$ bevorzugt aus den $C_{12}$ bis $C_{30}$ Kohlenwasserstoffketten, die Struktureinheit -$O-R_2$ aus den Ethylen- und/oder Propylenoxiden und n in einem Bereich von 15 bis 50 ausgewählt ist.

11. Copolymer nach einem der Ansprüche 9 oder 10, dadurch gekennzeichnet, daß die oberflächenaktive Verbindung vorzugsweise aus ethoxylierten Dilaurylaminen, ethoxylierten Octyl- und Nonylphenolen, oxyethylierten Laurin-, Stearyl-, Cetylalkoholen und ethoxylierten Mono-, Di- und Tristerylphenolen, einzeln oder als Gemisch ausgewählt ist.

12. Copolymer nach einem der Ansprüche 1 bis 11, dadurch gekennzeichnet, daß es, ausgedrückt in Gewichtsprozenten, enthält:

a) 15 bis 75%, und vorzugsweise 30 bis 45%, des oder der ethylenisch ungesättigten Monomeren mit wenigstens einer Carboxylfunktion,

b) 25 bis 70%, und vorzugsweise 45 bis 60%, des oder der anderen ethylenisch ungesättigten Monomeren ohne Carboxylfunktion,

c) 0,5 bis 35%, und vorzugsweise 4 bis 15%, des oder der oberflächenaktiven Monomeren/ mit einer Urethanfunktion, erhalten durch Reaktion eines ethylenisch ungesättigten Isocyanats mit einer oberflächenaktiven Verbindung, die eine mit der -NCO-Gruppe reaktionsfähige Hydroxylgruppe besitzt.

13. Copolymer nach einem der Ansprüche 1 bis 12, dadurch gekennzeichnet, daß es wenigstens teilweise neutralisiert ist.

14. Copolymer nach Anspruch 13, dadurch gekennzeichnet, daß das Neutralisierungsmittel vorzugsweise Lithium-, Natrium-, Kalium-, Ammonium-, Calcium-, oder Magnesiumhydroxid oder ein Amin ist, einzeln oder als Gemisch.

15. Wäßrige, gefüllte und/oder pigmentierte Zusammensetzungen, dadurch gekennzeichnet, daß sie das in den Ansprüchen 1 bis 14 definierte Copolymer enthalten.

16. Wäßrige, gefüllte und/oder pigmentierte Zusammensetzungen nach Anspruch 15, dadurch gekennzeichnet, daß sie, weiß oder gefärbt sind und als Hauptbestandteile eine wäßrige Phase, Füllstoffe und/oder Pigmente, ein natürliches oder synthetisches Bindemittel, das assoziative Verdickungscopolymere nach den Ansprüchen 1 bis 14, und gegebenenfalls als sekundäre Bestandteile ein Dispergierungsmittel und die verschiedensten Zusätze, wie Koaleszenzmittel, Biozide, oberflächenaktive und schaumhemmende Mittel enthalten.

17. Wäßrige Zusammensetzungen nach einem der Ansprüche 15 oder 16, dadurch gekennzeichnet, daß ihnen das verdickende Copolymer in einer Menge von 0,1 bis 10%, vorzugsweise 0,1 bis 5%, und besonders bevorzugt 0,4 bis 1,5% Trockengewicht, bezogen auf die Gesamtmenge der Zusammensetzungen, zugefügt wird.

18. Anwendungen des verdickenden Copolymeren nach einem der Ansprüche 1 bis 17 in wäßrigen Zusammensetzungen zur Beschichtung, Wasserfarben, Beizen zum Auftragen, Druckpasten, Produkten zur Lederverarbeitung, kosmetischen Zusammensetzungen, Reinigungsmitteln und Bohrflüssigkeiten.

## Claims

1. An associative thickening copolymer, soluble in neutral or alkaline aqueous medium, for white or coloured, filled and/or pigmented aqueous compositions, composed of:

a) at least one ethylenically unsaturated monomer having at least one carboxylic function,

b) at least one other ethylenically unsaturated monomer unprovided with a carboxylic function,

c) at least one surfactant monomer having at least one urethane function resulting from the reaction of an ethylenically unsaturated isocyanate with a surfactant compound having a hydroxyl function reactive towards the -NCO group,

characterized by the fact that the said copolymer belongs to the group of those which, by definition, when dissolved at a concentration of 2% by weight of dry matter in aqueous solution brought to a pH of 9 by addition of ammonia and at a temperature of 20 °C, have a maximum Brookfield viscosity at 100 rpm of 220 mPa.s (cP).

2. A copolymer according to Claim 1, characterized by the fact that the ethylenic monomer (a) having at least one carboxylic function is selected from the group consisting of the monoacids acrylic, methacrylic, crotonic and cinnamic, the diacids itaconic, fumaric, maleic and citraconic, maleic anhydride, and the $C_1$ to $C_4$ diacid hemiesters.

3. A copolymer according to Claims 1 or 2, characterized by the fact that the carboxylated ethylenic monomer is preferably acrylic acid, methacrylic acid or itaconic acid.

4. A copolymer according to any one of Claims 1 to 3, characterized by the fact that the ethylenically unsaturated monomer (b) unprovided with a carboxylic function is selected from the group consisting of methyl, ethyl, butyl, 2-ethylhexyl and lauryl acrylates and methacrylates; the acrylates and methacrylates of ethylene glycol, propylene glycol, poly(ethylene glycol) and poly(propylene glycol) as well as the corresponding phosphates and sulphates; acrylonitrile, acrylamide, N-(hydroxymethyl)acrylamide, dimethylaminoethyl acrylate and methacrylate, allyl alcohol, vinyl acetate, acrylamidomethylpropanesulphonic acid, styrene and methylstyrene.

5. A copolymer according to any one of Claims 1 to 4, characterized by the fact that the ethylenically unsaturated monomer (b) is preferably selected from the $C_1$ to $C_4$ acrylates and methacrylates.

6. A copolymer according to any one of Claims 1 to 5, characterized by the fact that the ethylenically unsaturated isocyanate entering into the composition of the surfactant monomer (c) results from the reaction of a diisocyanate with an ethylenic compound having a function reactive towards the -NCO group.

7. A copolymer according to Claim 6, characterized by the fact that the diisocyanate is selected from the group consisting of 1,4-diisocyanatobutane, 1,6-diisocyanatohexane, 2,2,4-trimethyl-1,6-diisocyanatohexane, 1,10-diisocyanatodecane, 4,4′-methylenebis(isocyanatocyclohexane), 1,4-cyclohexylene diisocyanate, 1-isocyanato-3-isocyanatomethyl-3,5,5-trimethylcyclohexane, m- and p-phenylene diisocyanates, toluene 2,4- and 2,6-diisocyanates, xylene diisocyanate, 4-chloro-1,3-phenylene diisocyanate, 4,4′-diphenylmethane diisocyanate, 1,5-naphthalene diisocyanate, and tetrahydronaphthalene diisocyanate.

8. A copolymer according to Claim 6, characterized by the fact that the ethylenic compound comprising a function reactive towards the -NCO group is selected from the group consisting of the acrylates and methacrylates of ethylene glycol, propylene glycol, poly(ethylene glycol) and poly(propylene glycol), allyl alcohol, allylamine, methallylamine and o-allylphenol.

9. A copolymer according to any one of Claims 1 to 8, characterized by the fact that the surfactant compound entering into the composition of the surfactant monomer (c) corresponds to the formula:

$$R_1\text{-(-O-}R_2\text{-)}_n\text{-OH}$$

in which:

– the unit ($O\text{-}R_2$) is ethylene oxide, propylene oxide, butylene oxide or a combination of at least two of these oxygenated groups,

– n, which represents the mean number of units present in the said surfactant compound, has a value between 5 and 150, and

– the unit $R_1$, comprising 1 to 32 carbon atoms, is selected from the group made up of hydrocarbon and/or amino chemical structures, such as aliphatic or cycloaliphatic alkyls, substituted or unsubstituted aryls, polyaryls, and secondary amines of formula $(R_3)(R_4)N$-, in which $R_3$ and $R_4$ are hydrocarbon groups comprising 1 to 20 carbon atoms.

10. A copolymer according to Claim 9, characterized by the fact that preferably the unit $R_1$ is selected from the $C_{12}$ to $C_{30}$ hydrocarbon chains, the unit $-O\text{-}R_2$ from ethylene oxide and/or propylene oxide and n from within the range 15 to 50.

11. A copolymer according to one of Claims 9 or 10, characterized by the fact that the surfactant compound is preferably selected from ethoxylated dilaurylamines, ethoxylated octyl- and nonylphenols, ethoxylated lauryl, stearyl and cetyl alcohols and ethoxylated mono-, di- and tristyryl phenols, alone or in a mixture.

12. A copolymer according to any one of Claims 1 to 11, characterized by the fact that it contains, expressed in weight percent:

a) from 15 to 75 %, and preferably from 30 to 45 %, of ethylenically unsaturated monomer(s) having at least one carboxylic function,

b) from 25 to 70 %, and preferably from 45 to 60 %, of other ethylenically unsaturated monomer(s) unprovided with a carboxylic function and

c) from 0.5 to 35 %, and preferably from 4 to 15 %, of surfactant monomer(s) having at least one urethane function resulting from the reaction of an ethylenically unsaturated isocyanate with a surfactant compound having a hydroxyl function reactive towards the -NCO group.

13. A copolymer according to any one of Claims 1 to 12, characterized by the fact that it is at least partly neutralized.

14. A copolymer according to Claim 13, characterized by the fact that the neutralizing agent is preferably lithium, sodium, potassium, ammonium, calcium or magnesium hydroxide or an amine, taken alone or in combination.

15. Filled and/or pigmented aqueous compositions, characterized by the fact that they contain the copolymer defined in Claims 1 to 14.

16. Filled and/or pigmented aqueous compositions according to Claim 15, characterized by the fact that, white or coloured, they contain as principal constituents an aqueous phase, fillers and/or pigments, a synthetic or natural binder, and the associative thickening copolymer according to Claims 1 to 14, and possibly as secondary constituents a dispersant and additives as various as coalescing agents, biocides, surfactants and antifoams.

17. Aqueous compositions according to one or other of Claims 15 or 16, characterized by the fact that the thickening copolymer is introduced to them at the rate of 0.1 % to 10 %, preferably of 0.1 % to 5 % and very preferably of 0.4 % to 1.5 % of dry weight in relation to the total weight of the said compositions.

18. Applications of the thickening copolymer according to any one of Claims 1 to 17 to aqueous coating compositions, water paints, aqueous coating slips, printing pastes, leather finishing products, cosmetic compositions, detergents and drilling fluids.